# EUROPEAN PATENT SPECIFICATION

(11) **EP 3 661 951 B1**
(45) Date of publication and mention of the grant of the patent: **07.08.2024**
(21) Application number: 18759402.3
(22) Date of filing: 30.07.2018
(51) Int. Cl.: C12P 23/00, C12N 9/02, C07K 14/415, C12N 15/82, C12N 15/52

(54) **GENES AND METHODS FOR BIOTECHNOLOGICAL PRODUCTION AND COMPARTMENTALIZATION OF HIGH ADDED VALUE APOCAROTENOIDS**
GENE UND VERFAHREN ZUR BIOTECHNOLOGISCHEN HERSTELLUNG UND KOMPARTIMENTIERUNG VON HOCHVEREDELTEN APOKAROTINOIDEN
GÈNES, PROCÉDÉS DE PRODUCTION BIOTECHNOLOGIQUE ET DE COMPARTIMENTATION D'APOCAROTÉNOÏDES À HAUTE VALEUR AJOUTÉE

(30) Priority: 03.08.2017 IT 201700089818; 03.08.2017 IT 201700089858
(43) Date of publication of application: 10.06.2020
(73) Proprietor: Agenzia Nazionale Per Le Nuove Tecnologie, L'Energia E Lo Sviluppo Economico Sostenibile (ENEA), 00196 Roma (IT); University of Zürich, 8006 Zurich (CH)
(72) Inventor: GIULIANO, Giovanni, 00165 Roma (IT); FERRANTE, Paola, 00165 Roma (IT); DIRETTO, Gianfranco, 00135 Roma (IT); APREA, Giuseppe, 00196 Roma (IT); DEMURTAS, Olivia Costantina, 00196 Roma (IT); PIETRELLA, Marco, 62010 Montefano (MC) (IT); MARTINOIA, Enrico, 00196 Roma (IT); FRANCISCO, Rita, 00196 Roma (IT)
(74) Representative: Di Giovine, Paolo
(86) International application number: PCT/IB2018/055669
(87) International publication number: WO 2019/025935

(56) References cited:
- WO-A2-2015/132411
- SHOIB AHMAD BABA ET AL: "Comprehensive transcriptome analysis of Crocus sativus for discovery and expression of genes involved in apocarotenoid biosynthesis", BMC GENOMICS, vol. 16, no. 1, 698, 15 September 2015 (2015-09-15), pages 1 - 14, XP055460001, DOI: 10.1186/s12864-015-1894-5
- DATABASE Protein [online] 1 March 2017 (2017-03-01), "ABC transporter C family member 14-like [Asparagus officinalis].", XP002779191, retrieved from NCBI Database accession no. XP_020276441
- TABASUM MOHIUDDIN ET AL: "Identification, phylogenetic analysis and expression profiling of ABC transporter family of Crocus sativus L: A step towards understanding apocarotenoid transport", PLANT GENE, vol. 14, 1 June 2018 (2018-06-01), pages 1 - 6, XP055513747, ISSN: 2352-4073, DOI: 10.1016/j.plgene.2018.02.001
- "Crocus sativus ALD9 polypeptide, SEQ:38", GENESEQ,, 5 November 2015 (2015-11-05), XP002779281
- S. FRUSCIANTE ET AL: "Novel carotenoid cleavage dioxygenase catalyzes the first dedicated step in saffron crocin biosynthesis", PROCEEDINGS OF THE NATIONAL ACADEMY OF SCIENCES, vol. 111, no. 33, 5 August 2014 (2014-08-05), pages 12246 - 12251, XP055208496, ISSN: 0027-8424, DOI: 10.1073/pnas.1404629111
- LOURDES GÓMEZ-GÓMEZ ET AL: "Unraveling Massive Crocins Transport and Accumulation through Proteome and Microscopy Tools during the Development of Saffron Stigma", INTERNATIONAL JOURNAL OF MOLECULAR SCIENCES, vol. 18, no. 12, 1 January 2017 (2017-01-01), pages 76, XP055460210, DOI: 10.3390/ijms18010076

## Description

### Technical field of the invention

The invention describes DNA sequences, vectors, proteins and use thereof for the biotechnological production of high added value saffron apocarotenoids and their transportation in a determined subcellular or extracellular compartment. The present invention in particular relates to genes, proteins and methods for producing saffron apocarotenoids in *in-vitro* systems, in engineered strains of *E. coli,* of yeast and in plant tissues.

### State of the art

*Crocus sativus L.* (Iridaceae) is a perennial, triploid, sterile plant, propagated and cultivated in temperate areas such as Spain or Kashmir. Its dried stigmas constitute the saffron spice which is commonly considered the most expensive spice in the world with prices varying from 2,000 to 7,000 €/Kg. The high prices of saffron are due to the huge manual work associated to the collection of stigmas: 1 Kg of dried saffron requires the manual collection of about 150,000 flowers (http://www.europeansaffron.eu/). Saffron stigmas accumulate a series of apocarotenoid compounds: crocetin and its glycosylated forms (crocins), responsible for the red colouring of stigmas, picrocrocin, responsible for its bitter flavour, and safranal, responsible for its pungent flavour. Apart from being high added value ingredients of food, the saffron apocarotenoid compounds have cosmetic and medical applications such as, by way of non-exhaustive example, the demonstrated protective function against retina's degenerative diseases of crocetin and crocins. Considering such applications and the high cost of the saffron apocarotenoids, which are currently produced though expensive extraction processes, there is an industrial interest in devising biotechnological methods for their low-cost production, through expression of the enzymes involved in their biosynthesis in microbial systems or in highly productive plants.

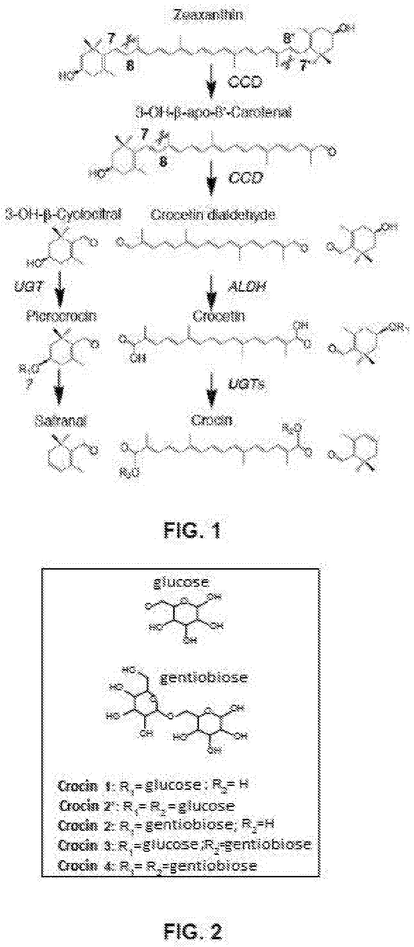

The biosynthesis of such compounds starts with the symmetrical cutting, in positions 7,8 and 7',8', of zeaxanthin thanks to CCD2 dioxygenase (see figure 1). The two cutting products of zeaxanthin, 3-OH-β-cyclocitral and crocetin dialdehyde, are dehydrogenated and glycosylated to produce picrocrocin and crocins, respectively. As far as the second biosynthetic step is concerned, dehydrogenation of crocetin dialdehyde into crocin, the patent application WO2013021261 and WO2015132411 show that it can be catalyzed into yeast from yeast endogenous aldehyde dehydrogenase (ALDH) or from exogenous dehydrogenase of various origin, including *Crocus sativus.*

However, neither of the two International patent applications shows data about the effectiveness therewith the describe ALDHs catalyze the reaction. Considering that the yeast does not synthetize crocetin dialdehyde and crocetin, and that *Crocus sativus* includes other aldehydes, apart from crocetin dialdehyde, likely to be the natural substrate of ALDHs shown in the patent applications, in order to obtain the biotechnological production of crocins it is necessary to detect ALDH(s) effectively catalysing the conversion of crocetin dialdehyde to crocetin in the stigmas of *Crocus sativus.*

Shoib Ahmad Baba et al. "Comprehensive transcriptome analysis of Crocus sativus for discovery and expression of genes involved in apocarotenoid biosynthesis" describes the transcriptome analysis of C.sativus to identify genes involve in the biosynthesis of carotenoids/apocarotenoids. In the study a number of enzymes involved in apocarotenoid biosynthesis were identified in comparing protein sequences present in transcriptome with those encoded by known genes.

The state of art as above reconstructed then highlights the need for providing new proteins, genes and methods for an effective production of apocarotenoids of *Crocus sativus L.,* in particular for the production of crocetin and crocin.

### Summary of the invention

The present invention is based upon the finding of methods and materials to improve the production of compounds of the saffron plant in recombinant systems, as well as nucleotides and polypeptides useful for producing compounds such as crocin and crocetin.

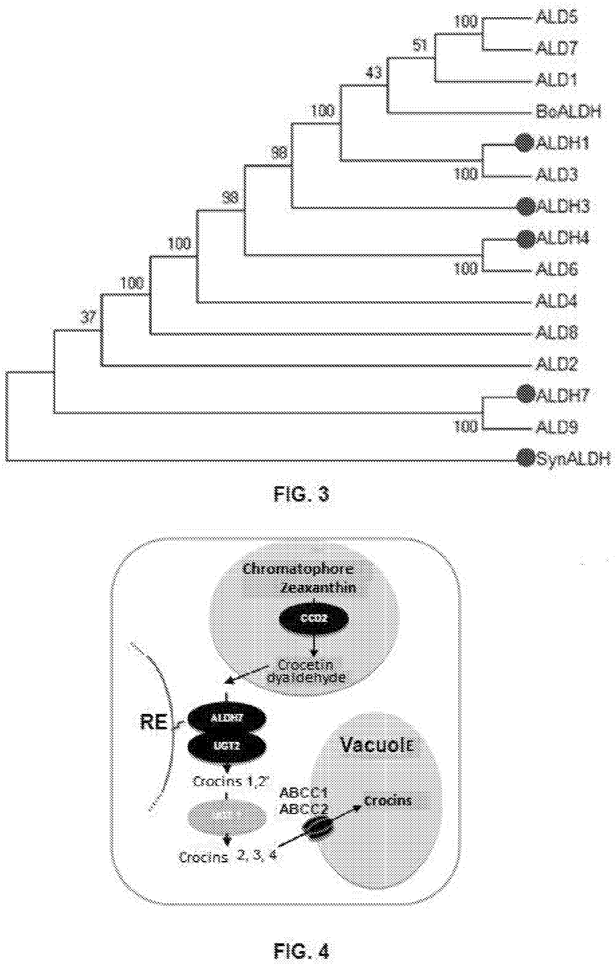

The inventors of the present patent application have surprisingly identified and characterized an aldehyde dehydrogenase expressed in the stigmas of *C. sativus* capable of catalyzing the conversion of crocetin dialdehyde to crocetin with high effectiveness. To this purpose, a bank of sequences expressed in the stigma of *C. sativus* was analysed by using *Blast* algorithm and a sequence of ALDH derived from *Bixa orellana,* which catalyzes the conversion of bixin dialdehyde to norbixin (GenBank: CAD70189.1). Different sequences of ALDH expressed in the stigma of *C. sativus* (CsALDH) were obtained by means of this analysis (SEQ ID NO 1-8).
ALDHs, at amino acid level, have different levels of identity with the ALDHs shown in the patent applications WO2013021261 and WO2015132411 and with the sequence of *Bixa orellana* used for their identification (see Table 1 and Figure 3). However, the experimental data produced by the inventors, and shown in the examples, show that only CsALDH7 enzyme having polypeptide sequence SEQ ID NO 8 is capable of converting crocetin dialdehyde to crocetin with high effectiveness.

**Table 1.**

| | **CsALDH1** (SEQ ID NO 2) | **CsALDH3** (SEQ ID NO 4) | **CsALDH4** (SEQ ID NO 6) | **CsALDH7** (SEQ ID NO 8) | **SynALDH** (SEQ ID Genbank: NP _442494.1) |
|---|---|---|---|---|---|
| **ALD1** | 78.31 | 37.47 | 27.05 | 25.57 | 26.87 |
| **ALD2** | 28.03 | 27.36 | 20.62 | 42.77 | 43.05 |
| **ALD3** | **100** | 38.90 | 26.38 | 27.63 | 26.91 |
| **ALD4** | 20.72 | 22.67 | 22.11 | 17.28 | 18.18 |
| **ALD5** | 74.90 | 38.49 | 27.94 | 26.54 | 28.34 |
| **ALD6** | 26.23 | 29.86 | **99.01** | 22.57 | 24.37 |
| **ALD7** | 74.30 | 37.40 | 27.44 | 27.82 | 26.70 |
| **ALD8** | 27.15 | 24.72 | 23.45 | 34.66 | 36.20 |
| **ALD9** | 24.60 | 27.31 | 20.36 | **99.79** | 43.42 |
| **BixaALDH** | 72.27 | 35.21 | 26.93 | 26.67 | 26.71 |

**Table 1**. Percentage of amino acid identity among the ALDHs described in the present patent application, the ALDs described in WO2013021261 and WO2015132411 and ALDH of *Bixa orellana* (in column BixaALDH). The identity percentages higher than 95% are shown in bolt. SynALDH is an ALDH of *Synechocystis* capable of converting apocarotenal and alcanal. Such ALDH can be expressed in a yeast, such as *Saccharomyces cerevisiae,* or in a bacterium such as *Escherichia coli,* engineered in order to accumulate crocetin dialdehyde through the expression of exogenous genes capable of catalyzing the synthesis of zeaxanthin and of CCD2 gene capable of converting zeaxanthin to crocetin dialdehyde. The expression of ALDH protein can be obtained by the use of DNA sequences capable of mediating high levels of expression in the selected organism, according to methods known to whoever is skilled in the art.

Moreover, the inventors based upon the activity data, the analyses of the hydrophobicity profiles and the alignments of the herein described CsALDH sequences succeeded in identifying the consensus sequences required to obtain ALDH capable of dehydrogenating aldehydes of apocarotenoids. More in details they have found that in order to obtain ALDH capable of dehydrogenating hydrophobic molecules such as aldehydes of apocarotenoids it is necessary that the enzyme comprises:
1) a partially hydrophobic region located upstream of the first site binding NAD(P) (herebelow an example is shown in underlined fonts) and of the first catalytic residue (N, in bolt) (sequences in which usually there are at least 90, more preferably 100-150 amino acids downstream of the protein beginning), having for example the following consensus sequence: TXXXXXXXXXEPXGXVLXISAW**N** (SEQ ID NO 21), wherein X is any amino acid residue, examples of such regions are the following sequences TFPSVGNIVAEPFGVVLIISAW**N** (SEQ ID NO 22), TFPSSAQIVSEPLGVVLVISAW**N** (SEQ ID NO 23), TYSMMNFRVKKEPLGTVLIIGPY**N** (SEQ ID NO 24).
2) A hydrophobic tail at the carboxy-terminus end preceded and followed by hydrophilic residues (shown in bolt), having for example the following consensus sequence: **KKXX**XXXALLXXNIXAXXLAFFGF**SKX** (SEQ ID NO 28), wherein X is any amino acid residue, examples of such hydrophobic tail are **KKRK**IIRALLAGNIIALVLAFFGFS**KS** (SEQ ID NO 25), KKKMVLKALLSSNIFAAILAFFGFS**KDS** (SEQ ID NO 26), **KGGVK**GALMRWLVVVAGYYLSAYMKA**RRA** (SEQ ID NO 27).

The inventors have further identified and characterized CsUGT2 enzyme (SEQ ID NO 10) which catalyzes the first steps of glycosylation of crocetin into crocins, by leading to the formation of crocin 1 and 2' (crocins containing 1 or 2 molecules of glucose, respectively). Such enzyme has a sequence identity of 90.5% with UGTCs2, an enzyme described in (Moraga et al., 2004, Planta, 219, 955-966) as responsible for all steps of glycosylation of crocetin. The inventors also identified and characterized two transporters of saffron, highly expressed in the stigmas, which are capable of carrying crocins through a lipid membrane. Such transporters belong to the family of ABC (ATP-binding cassette) membrane transporters, sub-family C. In the state of art, the use of ABC transporters to direct the accumulation of crocins in a determined cellular compartment is not described.

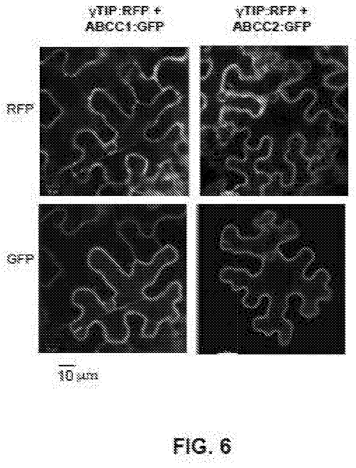

In order to identify the above-mentioned transporters, a bank of sequences expressed in the stigma of *Crocus sativus* was analyzed by using *Blast* algorithm and a sequence of ABC transporter, sub-family C (ABCC), derived from *Arabidopsis thaliana* (GenBank: AEC09006.1). The sequences of the transporters called CsABCC1 and CsABCC2 (SEQ ID NO 11-14) expressed in the stigmas of *Crocus sativus* were obtained through this analysis. Such ABCC transporters can be expressed in a yeast, such as *S. cerevisiae* or in a plant such as *Nicotiana benthamiana* engineered in order to accumulate crocins through the expression of exogeneous genes capable of catalyzing the zeaxanthin synthesis, of CCD2 gene capable of converting zeaxanthin to crocetin dialdehyde, one of ALDHs characterized by us and CsUGT2 capable of converting crocetin to crocins 1 and 2'. When expressed in *N. benthamiana,* CsABCC1 and CsABCC2 transporters show a tonoplastic location (vacuole membrane) (figure 6). In this way by expressing CsABCC1 or CsABCC2 transporters in engineered plants of *N. benthamiana,* it is possible to obtain the accumulation of crocins in the vacuole.

Based upon what said above the present invention relates to:
An amino acid sequence having a sequence selected between SEQ ID NO 12 (CsABCC1), SEQ ID NO 14 (CsABCC2) or an amino acid sequence with an identity percentage equal to or greater than 85% with SEQ ID NO 12 (CsABCC1) or SEQ ID NO 14 (CsABCC2), wherein said amino acid sequence is a transport protein apt to carry crocins through a lipid membrane.

A nucleotide sequence encoding a transport protein having amino acid sequence SEQ ID NO 12 (CsABCC1), SEQ ID NO 14 (CsABCC2) or an amino acid sequence with an identity percentage equal to or greater than 85% with SEQ ID NO 12 (CsABCC1) or SEQ ID NO 14 (CsABCC2).

An expression vector comprising:
- a nucleotide sequence encoding an aldehyde dehydrogenase having amino acid sequence SEQ ID NO 8 (CsALDH7) or an amino acid sequence with an identity percentage equal to or higher than 98% of SEQ ID NO 8, and/or
- a nucleotide sequence encoding an enzyme having amino acid sequence SEQ ID NO 10 (CsUGT2) or an amino acid sequence with an identity percentage equal to or higher than 91% of SEQ ID NO 10, and/or a nucleotide sequence encoding a transport protein having amino acid sequence SEQ ID NO 12 (CsABCC1), SEQ ID NO 14 (CsABCC2) or an amino acid sequence with an identity percentage equal or greater than 85% with SEQ ID NO 12 (CsABCC1) or SEQ ID NO 14 (CsABCC2).

A recombinant host organism or cell expressing:
- a transport protein having amino acid sequence SEQ ID NO 12 (CsABCC1), SEQ ID NO 14 (CsABCC2) or an amino acid sequence with an identity percentage equal to or greater than 85 % with SEQ ID NO 12 (CsABCC1) or SEQ ID NO 14 (CsABCC2).

A method for accumulating crocins in a determined sub-cellular compartment comprising a step of expression of a nucleotide sequence encoding a transport protein having amino acid sequence SEQ ID NO 12 (CsABCC1) or SEQ ID NO 14 (CsABCC2), optionally with target sequences specific for the addressing of a specific cellular or extracellular compartment, in particular wherein said subcellular compartment is the vacuole.

The present invention will be better understood by the following detailed description of some preferred embodiments thereof, provided with reference to the enclosed drawings, wherein the related Figures illustrate:

### Description of the figures

**Figure 1** **Biosynthetic route of apocarotenoids in saffron.** Biosynthetic route proposed for the biosynthesis of apocarotenoids. Zeaxanthin is cut in position 7,8 and 7',8' by the activity of a specific CCD of *C*. *sativus* (CCD2, Giuliano et al. Patent Application RM2014A000415, Frusciante et al., 2014, Proc Natl Acad Sci U S A, 111, 12246-12251). The cutting product C₂₀, crocetin dialdehyde, is converted to crocetin by an aldehyde dehydrogenase, then to crocins by the activity of at least two UDP-glycosyltransferase (UGT).
**Figure 2** **Glucose groups of most abundant crocins of *C*. *sativus* (crocins 1-4).**
**Figure 3** **Phylogenetic tree of the ALDHs described in the present patent application (designated with a black spot).** The tree was created by using the amino acid sequences with MEGA7 programme by applying the Neighbor-Joining method with bootstrap on 500 replications. The evolutive distances were calculated by using the p-distance method and they are represented in number of amino acid differences per site. The numbers shown on the branches designate the probability (in percentage) that this branch exists.
**Figure 4****. Model proposed for the biosynthesis/compartment of crocins of saffron.** CCD2 enzyme cuts zeaxanthin in the chromatophore, by producing crocetin dialdehyde which is transported in the cytoplasm. The conversion of crocetin dialdehyde to crocetin is performed by CsALDH7 enzyme which is anchored to a cytoplasmic membrane, probably the endoplasmic reticulum (RE) by means of its C-terminus hydrophobic tail and, together with CsUGT2 enzyme, it produces crocins 1 and 2 ' in the cytoplasm. A second UGT not yet identified converts the crocins 1 and 2 to crocins 2, 3 and 4. The crocins are then transported in the vacuole, through the action of the tonoplastic CsABCC1 and ABCC2 transporters.

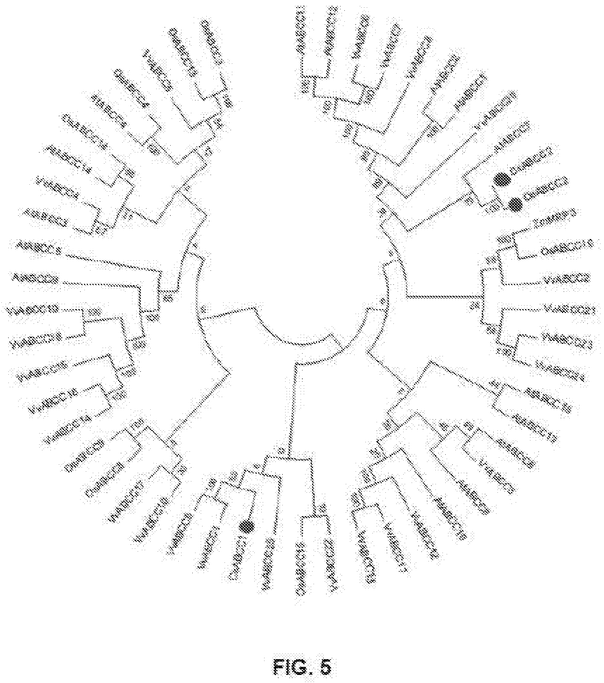

**Figure 5****. Phylogenetic tree of the ABCC transporters.** The tree was created with MEGA7 programme (Kumar S et al. Mol Biol Evol. [Epub ahead of print] PubMed PMID: 27004904) by applying the Neighbor-Joining method with bootstrap on 500 replications. The evolutive distances were calculated by using the p-distance method and they are represented in number of amino acid differences per site. The numbers shown on the branches designate the probability (in percentage) that this branch exists. The transporters of *C*. *sativus* characterized by us are designated by a black spot. Cs: *Crocus sativus;* Vv: *Vitis vinifera;* At: *Arabidopsis thaliana;* Os: *Oryza sativa.*
**Figure 6****.** Confocal microscope images of leaves of *N. benthamiana* expressing CsABCC1or CsABCC2 transporter fused to the green fluorescent protein (CsABCCI:GFP; CsABCC2:GFP) and the tonoplastic marker γTIP fused to the "red fluorescent protein" (yTIP:RFP). For each transporter the images of fluorescence of RFP and GFP are shown. It is observed that GFP signal, due to the expression of ABC transporters fused to GFP, co-locates with the signal of RFP, due to the expression of the tonoplastic marker γTIP, then showing that the two ABC transporters locate in the tonoplast.

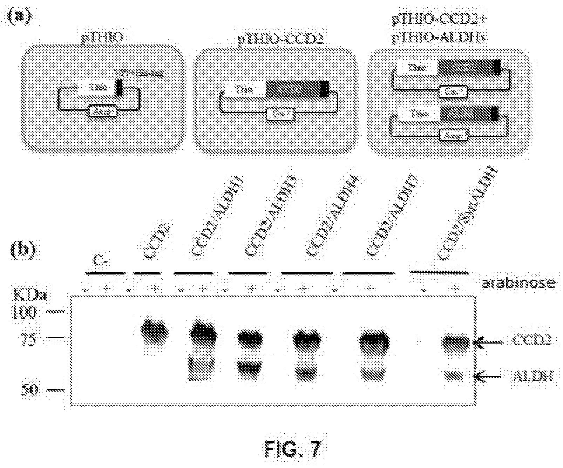

**Figure 7****. Expression of ALDHs in *E. coli.***
   **(a)** Schematic representation of the constructs expressed in the strain of *E. coli* which accumulates zeaxanthin. Each gene is inserted in pTHIO vector, fused at 5 ' to the gene of thioredoxin and at 3 ' of the sequence encoding a tag for purification (His6-tag). Cm^{r}: gene of resistance to chloramphenicol; Amp^{r}: gene of resistance to ampicillin.
   **(b)** Western blot performed with an antibody anti-His6 of the total proteins extracted from the recombinant bacterial strains before and after induction with arabinose (-) and (+). The arrows show proteins CCD2 and ALDH.

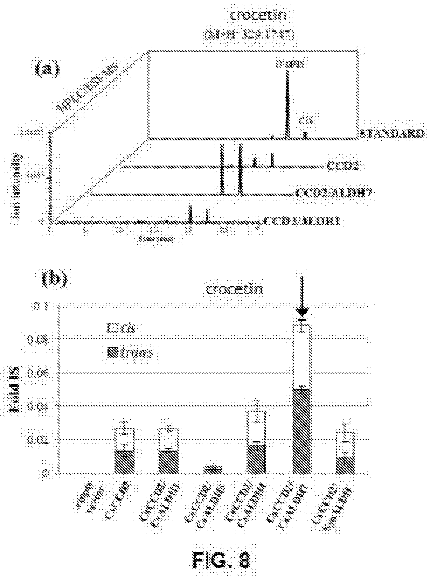

**Figure 8****. Accumulation of crocetin in the bacterial clones expressing CCD2 and ALDH enzymes**
   (a) Chromatograms HPLC-HRMS of crocetin (exact extracted mass of ion M+H⁺=329.1747) of the analytical standard (STANDARD) and of the bacterial clones which overexpress CCD2 enzyme alone (CCD2) or in combination with CsALDH7 or CsALDH1 enzymes (CCD2/CsALDH7; CCD2/CsALDH1). Two peaks of crocetin (isoform *trans* e *cis*) are noted.
   (b) Diagram of the crocetin levels (*trans* and *cis* isomers) accumulated in the recombinant bacterial clones. The results are shown as average values of three biological replications and the error bars represent standard deviations. Fold IS= fold internal standard (abundance with respect to the inner standard)

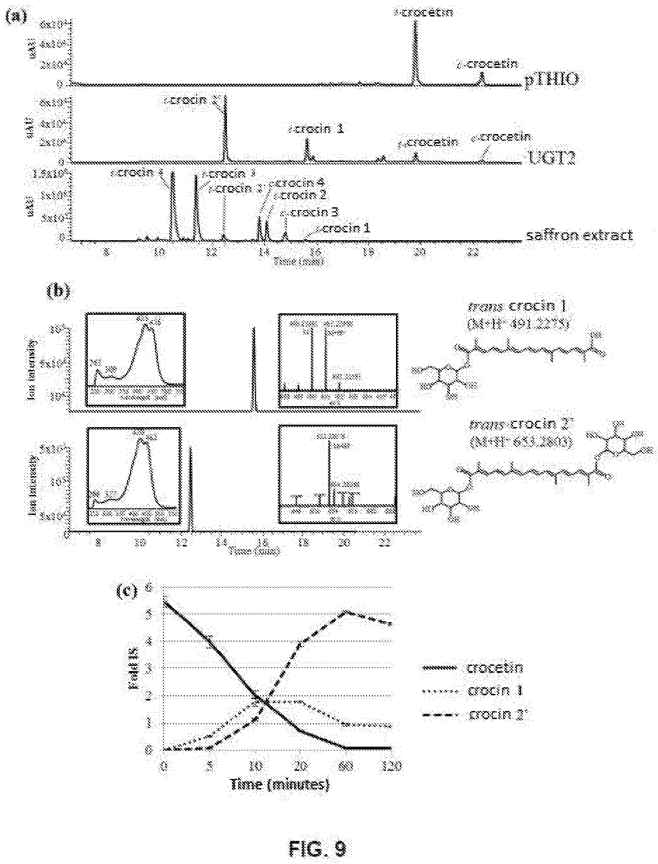

**Figure 9****. CsUGT2 enzyme catalyzes the conversion of crocetin to crocin 1 and crocin 2'.**
   **(a)** Spectra of absorption at 440 nm of the metabolites extracted from the reactions of *in vitro* assays performed for 60 minutes in absence (pTHIO) and in presence of CsUGT2 enzyme. Even the spectrum of the saffron extract is shown. t=*trans*; c=*cis.*
   **(b)** HPLC/HRMS (extracted exact masses) chromatograms of *trans* crocin 1 (M+H⁺=491.2275) and *trans* crocin 2' (M+H⁺=653.2803) which are accumulated in presence of the CsUGT2 enzyme. The boxes on the left of the peak of crocins show their spectrum of absorption, whereas the boxes on the right show the mass spectrum.
   **(c)** Time route of the conversion of crocetin to *trans* crocin 1 and *trans* crocin 2' in presence of CsUGT2 enzyme. The results are shown as average values of three biological replications and the error bars represent the standard deviations.

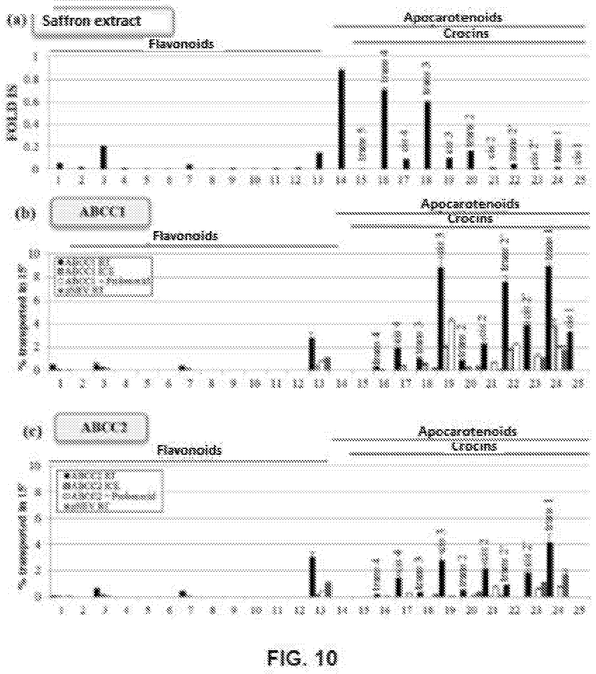

**Figure 10****. Transportation of saffron apocarotenoids by CsABCC1 and CsABCC2 transporters of *C*. *sativus.***
   (a) Related abundance (expressed as abundance with respect to the inner standard) of glycosylated metabolites identified in the saffron hydroalcoholic extract by LC-HRMS. 1: campherol 3-O-sophoroside-7 glucoside; 2: campherol 3,7,4'-triglucoside; 3: campherol 7-sophoroside; 4: campherol 3-β-D-glucopyranoside; 5: campherol 3-rutinoside-7-glucoside; 6: campherol acetylglucoside; 7: campherol -3-O-rutinoside; 8: dihydrocampherol -7-O-glucoside; 9: isorhamnetin-3-4'-diglucoside; 10: rhamnetin 3-rutinoside; 11: quercetin-3-diglucoside; 12: quercetin-3-O-rutinoside; 13: naringenin-7-O-glucoside (3 isoform); 14: picrocrocin; 15: trans-crocin 5; 16: trans-crocin 4; 17: cis-crocin 4; 18: trans-crocin 3; 19: cis-crocin 3; 20: trans-crocin 2; 21: cis crocin 2; 22: trans crocin 2'; 23: cis crocin 2'; 24: trans crocin 1; 25: cis crocin 1.
   (b) Net transport of saffron metabolites by transporters of *C*. *sativus* CsABCC1 and CsABCC2 and their pNEV control (empty vector). The transportation experiments were performed at room temperature (RT), on ice (ICE) and in presence of the specific inhibitor of the transporters ABCC, Probenecid (+ Probenecid). It is to be noted that at room temperature some metabolites, mainly crocins, are transported, whereas the reaction is inhibited if performed in ice or in presence of Probenecid inhibitor. The net transport was calculated by deducting the values measured in absence of ATP from the values measured in presence of ATP after 15 minutes of transportation.
The results are shown as average values of three biological replications and the error bars represent standard deviations.

### Detailed description of the invention

The present invention will be better illustrated with reference to the following Embodiment examples, which are provided by way of illustration and not as limiting example.

It is herein disclosed, but not part of the present invention, an amino acid sequence having SEQ ID NO 8 (CsALDH7), wherein said amino acid sequence SEQ ID NO 8 is an aldehyde dehydrogenase apt to catalyze the conversion of crocetin dialdehyde to crocetin with high effectiveness. Are herein disclosed, but not part of the present invention,amino acid sequences with an identity percentage equal to or higher than 98% with SEQ ID NO 8, preferably equal to or higher than 99%. Such sequences for example could have conservative mutations with respect to SEQ ID NO 8 which substantially do not alter the effectiveness and the catalytic specificity of the enzyme.

It is herein disclosed, but not part of the present invention, an amino acid sequence having SEQ ID NO 10 (CsUGT2), wherein said amino acid sequence CsUGT2 is an enzyme capable of catalyze glycosylation of crocetin into crocin 1 and 2'. It is also disclosed herein, but not part of the present invention, amino acid sequences with an identity percentage equal to or higher than 91% of SEQ ID NO 10, preferably higher than 92%, 95% or than 99% of identity.

The present invention further relates to the polypeptides having a sequence selected from SEQ ID NO 12 (CsABCC1) and SEQ ID NO 14 (CsABCC2), wherein said amino acid sequences SEQ ID NO 12 (CsABCC1) and SEQ ID NO 14 (CsABCC2) are transport proteins capable of transporting crocins through a lipid membrane. The present invention also relates to amino acid sequences with an identity percentage equal or greater than 85 %, preferably higher than 90%, 95% or 99% of identity with respect to SEQ ID NO 12 (CsABCC1) or to SEQ ID NO 14 (CsABCC2). Such sequences for example could have conservative mutations with respect to SEQ ID NO 12 (CsABCC1) and SEQ ID NO 14 (CsABCC2) which do not alter the specific protein transport function.

The herein described amino acid sequences can include additional amino acids not involved in the specific enzymatic or transport activity performed by the protein and then such sequences for example could further include a purification tag (for example tag HIS or tag GST) or sequences which stabilize the protein (for example thioredoxin used in the constructs for the expression of ALDH enzymes in *E. coli,* Figure 7a). In some embodiments, the herein described amino acid sequences for example could include a sequence of amino acids acting as reporter, for example GFP (Green fluorescent protein). The percentage of identity for any nucleic acid or polypeptide (amino acid sequence) to a reference nucleic acid or polypeptide can be determined as it is known to the person skilled in the art. A reference sequence (for example a sequence of nucleic acid or a sequence of amino acids) is aligned to one or more sequences of candidates by using Clustal Omega program (http://www.ebi.ac.uk/Tools/msa/clustalo/, predefined parameters) allowing to perform alignments of sequences of nucleic acid or polypeptides along the whole length thereof (global alignment). See Chenna et al., Nucleic Acids Res., 31 (13): 3497-500 (2003*).*

The present invention also relates to the following nucleotide sequences:
the nucleotide sequences codifying for a transport protein having amino acid sequence SEQ ID NO 12 (CsABCC1), SEQ ID NO 14 (CsABCC2), for example nucleotide sequence SEQ ID NO 11 or SEQ ID NO 13.

According to an embodiment synthetic nucleotide sequences could be used, for example by using codons optimized according to the selected recombinant host organism or cell. Due to the degeneration of the genetic code, for many amino acids there is more than a triplet of nucleotides for coding the same amino acid. Then, the codon in the coding sequence for a determined polypeptide can be modified so as to optimize the expression in a particular host organism, by using the methods known to the person skilled in the art. According to an embodiment even all cDNAs of the herein described nucleotide sequences belong to the present invention.

The present invention further relates to expression vectors, recombinant host cells and organisms expressing one or more of the herein described amino acid sequences. The expression vectors known to the person skilled in the art for expressing the involved enzyme or protein suitable for the specific expression system could be used. The expression vectors can be found commercially or can be constructed *ad hoc* both for the expression in microbial and plant systems. In order to express the proteins of *C*. *sativus* in bacterial cells as not exhaustive example vectors pTHIO-Dan1 (shown in figure 7a), vectors pQE (Qiagen), vectors for the series pET (Invitrogen) can be used, whereas the vectors for yeast expression can be for example pNEV (used in figure 10), pAG (Add gene), pYES (Thermo Fisher). For the expression of the enzymes and of the transporters in plant for example vectors derived from pBI121 (Clontech), or from pCAMBIA (Gambia) can be used.

Such vectors could comprise apart from the herein described nucleotide sequences a (prokaryotic and eukaryotic) "regulatory region", that is a sequence of nucleotides influencing the transcription or translation and the speed, the stability and/or mobility of a transcription or translation product. The regulatory regions for example include sequences of promoters, sequences of potentiation, response elements, sites for recognizing the proteins, inducible elements, binding sequences of proteins, regions not translated by 5' and 3' (UTR), introns and combination thereof. The regulatory region will be operatively bound to the nucleotide sequences codifying one or more proteins of the present invention.

The recombinant cells and organisms including the herein described nucleotide sequences or expression could be without any limitation all expression systems known to the person skilled in the art suitable to express one or more involved amino acid sequences, for example SEQ ID NO 8 (CsALDH7, that is not part of the invention), SEQ ID NO 10 (CsUGT2, that is not part of the invention) SEQ ID NO 12 (CsABCC1), SEQ ID NO 14 (CsABCC2).

According to an embodiment the recombinant cells and organisms express one or more of recombinant proteins having SEQ ID NO 12 (CsABCC1), SEQ ID NO 14 (CsABCC2), together with CCD2 enzyme which cuts zeaxanthin in position 7,8 and 7',8'. According to a preferred embodiment it will be a host recombinant cell or organism accumulating zeaxanthin.

Anyone of the herein described host organisms can be a microorganism, a plant or a plant cell. The microorganism can be for example a yeast such as *Saccharomyces cerevisiae* or a bacterium such as *Escherichia coli.* The plant or plant cell can be for example *Arabidopsis thaliana, Nicotiana benthamiana, Zea mays, Solanum lycopersicum, Solanum tuberosum, Oryza sativa.*

A herein described recombinant host cell can be a yeast cell, a plant cell, a mammal cell, an insect cell, a bacterial cell. In some embodiments, the yeast cell is a cell of *Saccharomyces cerevisiae, Schizosaccharomyces pombe, Yarrowia lipolytica, Candida glabrata, Ashbya gossypii, Cyberlindnera jadinii, Pichia pastoris, Kluyveromyces lactis, Hansenuia poiymorpha, Candida boidinii, Arxula adeninivorans, Candida albicans.*

In some embodiments, the nucleic acids and the herein described polypeptides are inserted in plants. Therefore, a host can be a plant or plant cell including at least a herein described recombinant gene. A plant cell or plant can be transformed having a recombinant gene integrated in its genome, that is it can be transformed stably. The cells transformed stably typically keep the nucleic acid inserted with each cell division. A plant cell or plant can even be transformed transitory so that the recombinant gene is not integrated in its genome. The transgenic plant cells used in the herein described methods can constitute a portion or a complete plant. Techniques for the insertion of nucleic acids in monocotyledon and dicotyledon plants are known in the state of art and they include for example the transformation mediated by *Agrobacterium* and electroporation.

According to an embodiment the recombinant cells and organisms are prepared so as to overexpress enzymes leading to the accumulation of the precursor of the biosynthetic route of apocarotenoids in saffron (zeaxanthin) such as for example *crtE* (Genbank ALK24264.1), *crtYB* (Genbank AAO73816.1), *crtI* (Genbank ALK24268.1) genes of the yeast *Xanthophyllomyces dendrorhous* and *crtZ* of the bacterium *Erwinia uredovora* (Genbank ADD79330.1).

The recombinant host microorganisms could be prepared by transforming with the nucleotide sequences, for example with the above-described vectors, according to the known techniques, so as to express the herein described involved proteins.

It is also disclosed but not part of the present invention a method for producing crocetin and/or crocin in a host organism or cell accumulating zeaxanthin, comprising a step of expression in said organism of the enzyme having amino acid sequence SEQ ID No. 8 (CsALDH7) and/or SEQ ID N. 10 (CsUGT2). It is also disclosed but not part of the present invention an embodiment wherein the recombinant host organism or cell is transformed with the herein described nucleotide sequences, for example SEQ ID N. 7 (CsALDH7) and/or SEQ ID N. 9 (CsUGT2), so as to over-express the involved enzymes.

The present invention further relates to a method for accumulating crocins in a determined subcellular compartment of a recombinant host organism comprising a step of expression of a nucleotide sequence encoding a transport protein having amino acid sequence SEQ ID NO 12 (CsABCC1) or SEQ ID NO 14 (CsABCC2).

In such methods anyone of the herein described recombinant organisms or cells, in particular *Saccharomyces cerevisiae eNicotiana benthamiana* transformed with one of the herein described sequences or vectors could be used, so as to over-express the involved amino acid sequences. Under the term "over-express" it is meant that the recombinant organisms express in higher amount the exogenous gene with respect to the possible corresponding endogenous gene.

It is herein disclosed, but not part of the present invention a method for dehydrogenating an aldehyde of apocarotenoid, in particular for dehydrogenating crocetin dialdehyde into crocin, comprising a step for dehydrogenating said aldehyde with an aldehyde dehydrogenase comprising:
1) a partially hydrophobic region located after at least 90 residues from the amino-terminus end, preferably after 100-150 residues, and before the binding site of nicotinamide adenine dinucleotide phosphate (NAD(P));
2) a hydrophobic tail located at the C-terminus end preceded and followed by one or more hydrophilic residues, for example by two, three, four residues.

It is also disclosed but not part of the present invention an embodiment wherein said partially hydrophobic region has the following consensus sequence:
TXXXXXXXXXEPXGXVLXISAW**N** (SEQ ID NO 21), wherein X is any amino acid residue. Examples of such regions are the following sequences TFPSVGNIVAEPFGVVLIISAW**N** (SEQ ID NO 22), TFPSSAQIVSEPLGVVLVISAW**N** (SEQ ID NO 23), TYSMMNFRVKKEPLGTVLIIGPY**N** (SEQ ID NO 24).

It is also disclosed but not part of the present invention an embodiment wherein said hydrophobic tail has the following consensus sequence:
**KKXX**XXXALLXXNIXAXXLAFFGF**SKX** (SEQ ID NO 28), wherein X is any amino acid residue.

Examples of such hydrophobic tail are the following sequences **KKRK**IIRALLAGNIIALVLAFFGFS**KS** (SEQ ID NO 25), **KKKM**VLKALLSSNIFAAILAFFGFS**KDS** (SEQ ID NO 26). ,**KGGVK**GALMRWLVVVAGYYLSAYMKA**RRA** (SEQ ID NO 27).

It is also disclosed but not part of the present invention an aldehyde dehydrogenase having at the carboxy-terminus end the amino acid sequence **KGGVK**GALMRWLVVVAGYYLSAYMKA**RRA** (SEQ ID NO 27) or an amino acid sequence with 80% of identity preferably with 85%, 90%,95%, 99% of identity with SEQ ID NO 27. Moreover, the present invention also relates to SEQ ID NO 27 on itself and amino acid sequences con 1'80% of identity preferably with 85%, 90%, 95%, 99% of identity with this sequence.

It is also disclosed but not part of the present invention a method for evaluating a transporter's capability of transporting a determined metabolite, herein designated assay of "transportomics", comprising the following steps:
- incubating a preparation comprising a transporter with a complex plant extract comprising a plurality of metabolites;
- detecting the amount of metabolite which was transported.

It is also disclosed but not part of the present invention an embodiment wherein the preparation used in the method is constituted by microsomes of yeast including the transporter to be characterized.

It is also disclosed but not part of the present invention an embodiment wherein the plant extract will be a hydroalcoholic extract or an extract obtained with apolar solvents from organs or tissues, for example of *Crocus sativus* or other involved plants.

It is also disclosed but not part of the present invention a method for dehydrogenating the retinal or other apocarotenals (aldehyde of apocarotenoids), with a number of carbon atoms higher than 20 comprising a step of dehydrogenating said aldehydes with an aldehyde dehydrogenase having SEQ ID NO 8 (CsALDH7).

It is also disclosed but not part of the present invention a method for the conversion of apocarotenals with a number of carbon atoms greater than or equal to 20 in the corresponding carboxylic acid in a host organism or cell capable of accumulating the above-mentioned apocarotenals, comprising a step of expression in said organism or cell of the enzyme having amino acid sequence having SEQ ID NO 8 (CsALDH7). It is also disclosed but not part of the present invention an embodiment wherein the host organism is a bacterium, a yeast or a recombinant plant, for example selected from *Saccharomyces cerevisiae, Schizosaccharomyces pombe, Yarrowia lipolytica, Candida glabrata, Ashbya gossypii, Cyberlindnera jadinii, Pichia pastoris, Kluyveromyces lactis, Hansenula polymorpha, Candida boidinii, Arxula adeninivorans, Xanthophyllomyces dendrorhous, Candida albicans species, Escherichia coli, Arabidopsis thaliana, Nicotiana benthamiana, Zea mays, Solanum lycopersicum, Solanum tuberosum, Oryza sativa.*

### Examples

### Reference Example 1: Expression of different ALDHs in E. coli Bacterium.

The genes codifying for ALDH were insulated starting from RNA of saffron with specific oligonucleotides. The genes were then cloned in a vector for the expression in a bacterium having a resistance to antibiotics different from the vector containing CCD2 (methods described in 5,6). Such vectors were introduced in a strain of *E. coli* capable of producing zeaxanthin and containing a vector expressing CCD2, and then capable of producing crocetin dialdehyde (Giuliano et al. Patent Application RM2014A000415, Frusciante et al Proc Natl Acad Sci U S A 111 (33):12246-12251). The so-obtained bacterial strains, each one expressing both CCD2 gene and one of ALDH genes (figure 7a), were grown under conditions leading to the expression of proteins CCD2 and ALDH (Giuliano et al. Patent Application RM2014A000415, Frusciante et al Proc Natl Acad Sci U S A 111 (33):12246-12251). As checks the strains including the empty vector or the vector including CCD2 gene (figure 7a) were used. The analysis of the correct protein expression was performed by means of SDS-PAGE followed by western blot (figure 7b) performed as described in Demurtas et al. Front Plant Sci. 7:54).

The so-obtained microorganism can be analyzed by one of the methods known by the person skilled in the art to detect the presence and quantity of zeaxanthin, crocetin dialdehyde and crocetin.

### Reference Example 2: Identification and quantization crocetin in a E. coli bacterium expressing different ALDHs.

The production of crocetin and the quantification of the produced levels were analyzed on extracts obtained from the recombinant clones obtained by liquid chromatography coupled to high resolution mass spectrometry (LC-HRMS). After the induction of CCD2 and ALDH proteins, the cells were collected by centrifugation and resuspended in specific solvents for the extraction of polar and apolar metabolites. The polar metabolites extracted with methanol 75% were separated with a chromatographic column C18 Luna of reversed phase (150 × 2.1 mm, 3µm; Phenomenex) and ionized by means of electrospray (ESI source) (Fasano et al. New Phytologist 210: 1382-1394). On the contrary the non-polar metabolites were extracted with acetone, separated with chromatographic column C30 with reversed phase (100 × 3.0 mm; YMC Europe) and ionized by chemical ionization at atmospheric pressure (APCI source).

The mass corresponding to the ion deriving from crocetin (M+H⁺=329.1747) was extracted from chromatograms and compared to that of the authentic standard (figure 8a). The produced crocetin exists in the form of 2 isomers: *trans* e *cis.* The levels of crocetin *trans* and *cis* accumulated by each recombinant bacterium strain are shown in figure 8b.

### Example 3: Expression of ABCC transporters of C. sativus in microsomes of S. cerevisiae.

The genes codifying for CsABCC1 and CsABCC2 transporters were insulated starting from RNA of saffron with specific oligonucleotides. The genes then were cloned in a vector for the expression in *Saccharomyces cerevisiae,* pNEV-Ura, and expressed in a strain of *S*. *cerevisiae,* defective for ABCC transporter "Yeast Bile Transporter1" (YBT1). As check, the same strain was transformed with the empty pNEV vector.

From the transformed strains of yeast microsome preparations were performed, that is the total vesicles of the cellular endomembranous system were extracted as described in Tommasini et al. Proc Natl Acad Sci USA. 93(13):6743-6748. Such vesicles include ABCC transporter to be characterized.

In order to evaluate the physiological integrity of the insulated vesicles, we analyzed the capability of transporting the substrate leukotriene C4 (LTC4) (Leier et al., 1994, J Biol Chem 269(45): 27807-10).

### Example 4: Development and use of an in vitro assay for characterizing the functionality and studying specificity of ABCC transporters of C. sativus.

A new *in vitro* assay was prepared, differing from the classical transportation assay wherein the vesicles are left to incubate for pre-established periods of time with the putative substrate, washed, lysed and the content analysed by liquid chromatography coupled to a UV/visible detector. With respect to the works existing in literature relating the in-vitro functional characterization of transporters, the present invention introduces the concept of "transportomics": the assay is based upon the transportation analysis, performed by LC-HRMS, of all molecules existing in the saffron extract. This approach, providing the use of a raw extract instead of commercial purified standards, allows to characterize the transportation even of molecules which are not commercially available; moreover, as it is based upon data obtained with high resolution mass spectrometry, rather than with photodiode series detector (PDA), the system is extremely sensitive and it allows to appreciate the transportation of low abundant metabolites.

Figure 10a shows the content of the main glycosylated metabolites existing in the saffron hydroalcoholic extract, whereas figure 10b shows the metabolites which are specifically transported by CsABCC1 and CsABCC2 transporters of *C*. *sativus.*

With this method, then, it was observed that the two ABCC transporters are capable of transporting different crocins apart from some flavonoids (figure 10b, black bars). Such transportation is not due to a simple spreading of the substrates but it is due to the action of an enzyme since upon performing the assay in ice (wherein enzymatic activity is reduced) transportation reduction is noted (figure 10b, punctated white bars). Moreover, the transportation is due to the activity of ABCC transporters, since by adding Probenecid inhibitor (an inhibitor of ABC transporters) a considerable transportation reduction is obtained (figure 10b, white bars).

### Experimental procedures:

### Insulation of genes of C. sativus.

The genes CsALDH, CsUGT2, CsABCC1 and CsABCC2 were insulated by RNA extracted from saffron stigmas collected on the day before the anthesis and coming from the firm Zafferanami, Varedo (MI) (http://zafferanami.tumblr.com/). In details, RNA was retrotranscribed in cDNA by means of kit Omniscript RT cDNA synthesis kit (Qiagen). The codifying sequences (CDS) of the genes were obtained by amplifying cDNA with Phusion High Fidelity DNA polymerase (NEB) with pairs of oligonucleotides specific for each gene:
CsALDH1: ATGGCTGCAACTAACAGCAATG(SEQ ID NO 15); TCACAGCCAAGGTGAGTTATAT *(SEQ ID NO 34)*
CsALDH3: ATGTCGATGCTACGCGTGG(SEQ ID NO 16); TCAAGACTCCAAGTTCCCC *(SEQ ID NO 33)*
CsALDH4: ATGGGATTTACCAAGGAGCAC *(SEQ ID NO 35);* CTAGCCAAAGTTTATTCCCTG *(SEQ ID NO 36)*
CsALDH7: ATGGCCTTCGATGGAGAGAAAG (SEQ ID NO 17); TCAAGATTTGGAGAAGCCAAAG (SEQ ID NO 18)
CsUGT2: ATGGAGCAGAAAGATGTGAA(SEQ ID NO 19); TCATTTGCAACACTGATGTATG (SEQ ID NO 32)
CsABCC 1: ATGTCCTCCTCTCCCTAC(SEQ ID NO 20)
; TCAAAGATCAGAAGAGCGGTT () (SEQ ID NO 31)
CsABCC2: ATGGGTTTCAAACCGCTTG(SEQ ID NO 29); CTACATTTCAGCATTGTCC) (SEQ ID NO 30)

The obtained amplicons were then cloned in vectors for the expression in bacterium, yeast and plant.

### Functional characterization of ALDH enzymes by means of in vivo assay in bacterium.

In order to assay the enzymatic activities of various ALDHs the genes were cloned in pTHIO vector (containing the gene for the resistance to ampicillin, Amp^{r}) (Trautmann et al., 2013, Febs j, 280(15): 3685-96) for the expression in bacterium and the obtained constructs were inserted by means of electroporation in a strain of *E. coli* accumulating zeaxanthin (containing the gene for the resistance to kanamycin, Kan^{r}) described in (Frusciante et al 2014, Proc Natl Acad Sci U S A 111 (33):12246-12251) and bearing pTHIO-CCD2 plasmid (containing the gene for the resistance to chloramphenicol, Cm^{r}) (figure 7a). As check the strain bearing only CCD2 gene or bearing empty pTHIO plasmid was used. The so-obtained recombinant bacteria were grown at 37°C in liquid medium LB containing antibiotics with half concentration (25 µg/ml kanamycin, 12.5 µg/ml chloramphenicol and/or 50 µg/ml ampicillin), until reaching an optical density at 600 nm of 0.7. The expression of proteins was then induced with 0.08% arabinose at 20 °C for 16 hours. The cells were then collected by centrifugation and the bacterial pellets re-suspended in solvents specific for extracting the polar/half-polar and non-polar metabolites. The polar/half-polar metabolites were extracted as described in (Fasano et al. New Phytologist 210: 1382-1394) with small modifications: the pellets coming from 50 ml of liquid culture were re-suspended in 10 mL of cold methanol including 0.5 µg/ml formononetin, Sigma-Aldrich,), lysed in ice by sonication and centrifuged at 18,000 g for 30 minutes. The supernatant was dried and re-suspended in 200 µL of 75% methanol, further centrifuged for 20 minutes for removing possible aggregated materials and precipitated and subjected to HPLC-HRMS analysis. The non-polar metabolites were extracted with the same procedure, by using acetone including 0.5 µg/ml of α-tocopherol acetate for the extraction and chloroform for the re-suspension.

### Functional characterization of CsUGT2 enzyme by in vitro assay.

In order to assay the enzymatic activity of CsUGT2 the gene was cloned in pTHIO vector containing the gene for the resistance to spectinomycin, Spect^{r}) and the obtained construct was inserted by electroporation in the strain of *E. coli* BL21(pGro7) described in (Frusciante et al 2014, Proc Natl Acad Sci U S A 111 (33): 12246-12251). The expression of CsUGT2 protein was induced as described for ALDH. An *in vitro* assay was then developed by using the raw lysate of the bacteria overexpressing the obtained CSUGT2 enzyme by using the buffer LEW as described in (Frusciante et al 2014, Proc Natl Acad Sci U S A 111 (33): 12246-12251). The assay was performed in 100 µl of reaction by using the bacterial lysate containing 40 µg of total proteins, the substrate crocetin (100 µM) incapsulated in 6-O-α-maltosyl-β-cyclodextrins and UDP-glucose (2.5 mM) substrate as described in (Moraga et al., 2004, Planta, 219, 955-966). The reaction was performed at 30°C and stopped at different periods of time (0, 5, 10, 20, 60, 120 minutes) by adding 100 µl of cold ethanol and freezing at -20°C. The polar/half-polar metabolites were extracted by sonication and centrifugation as described for ALDHs.

### Characterization of subcellular localization of ABC transporters.

CsABCC1 and CsABCC2 transporters were fused at the carboxy-terminus end with the "enhanced Green Fluorescent Protein" (eGFP) and cloned in pBI121 vector (Clontech). As tonoplastic marker γ-TIP protein fused to "Red Fluorescent Protein" (RFP) was used (Nelson, et al, 2007 Plant J 51(6): 1126-36). The constructs were inserted by electroporation in C58C1 strain of *Agrobacterium tumefaciens* and the recombinant bacteria were used to transform in a transient way (agroinfiltration) leaves of *Nicotiana benthamiana* as described in (Hamilton & Baulcombe, 1999, Science 286(5441): 950-2). The leaves were agroinfiltrated contemporary to the construct bearing ABC transporter and with the tonoplastic marker. 48 hours after infiltration the leaves were observed with a confocal laser microscope (Olympus FV1000). Lasers at 488 nm (Ar) and 635 nm (diode) were used to display fluorescence of GFP and RFP, respectively.

### Functional characterization of ABC transporters by in vitro assay.

In order to assay the activity of CsABCC1 and CsABCC2 transporters, the genes were cloned in pNEV vector (Sauer & Stolz, 1994, Plant J 6(1): 67-77) for yeast expression. The obtained constructs were inserted by electroporation in ybt1 mutant of *S. cerevisiae* (MATa; ura3D::HIS3; leu2-3, 112; his3-D200; bat1D1::URA3). As check, the strain was transformed with empty pNEV vector. From obtained transformants microsomal fractions were prepared as described in (Tommasini et al. Proc Natl Acad Sci U S A. 93(13):6743-6748). The integrity of insulated vesicles was evaluated by analysis of the ability to transport the substrate leukotriene C4 (LTC4 (Leier et al., 1994, J Biol Chem 269(45): 27807-10). The transportation assays of the saffron metabolites were performed by using the technique of quick filtration (Tommasini et al. Proc Natl Acad Sci U S A. 93(13):6743-6748) with filters of cellulose acetate (size of pores: 0.45µm). The devised assay of "transportomics" provides, instead of analytical standards, the use of saffron hydroalcoholic extract as substrate. Such extract was prepared by pulverizing 3 mg of dried stigmas (provenance, Zafferanami), by resuspending in 300 µl of cold 50% methanol and by homogenizing for 40 minutes in Mixer Mill 300 at 20 Hz frequency and at last by centrifuging for 20 minutes at 20000 g. The recovered supernatant was characterized by HPLC-HRMS and standardized amounts used in the transportation assays (total content of crocins equal to 320 µM). The transportation reaction was performed in a final volume of 650 µL by using 100 µL of microsomal preparation (having optical density at 600 nm of 4 and a total protein content of 400 µg), in transportation buffer (0.4 M glycerol, 0.1 M KCl, 20 mM Tris-MES, pH 7.4) then adding 1 mM DTT, 6 mM or 1 mM MgSO₄ (in presence or absence of MgATP, respectively), 100 µg/mL of creatine kinase and 10 mM of creatine phosphate. The transportation assay was performed in presence or absence of 4 mM MgATP. The assay was performed at room temperature and stopped after 30 seconds and after 15 minutes. The inhibition assays were performed by incubating the microsomes at room temperature in the transportation mix containing Probenecid inhibitor (1 mM) for 10 minutes and then adding the saffron extract. The assays of inhibition in ice were performed by incubating in ice the tubes containing the reaction mix, immediately after the addition of microsomes. All transportation reactions were stopped by filtration of 100 µl of reaction mix and washing of filters with 2.5 mL of cold transportation buffer for three times. The vesicles collected on the filters were dissolved and the metabolites extracted by incubating the filters in 1 ml of 75% methanol and putting under stirring for 20 minutes. The lipids of vesicles were removed by collecting 800 µl of sample, by adding 400 µl of chloroform, by stirring by vortex and Mixer Mill for 5 minutes at frequency of 20 Hz. 200 µl of ultrapure water were then added and the steps were separated by stirring and centrifugation at 20000 g for 20 minutes. The upper phase was collected, dried and re-suspended in 80 µl of 50% methanol containing 0.5 µg/ml of formononetin and analyzed by HPLC-HRMS.

### HPLC-HRMS analysis.

The samples were analyzed by means of a Q-exactive mass spectrometer (ThermoFisher Scientific), coupled to HPLC system provided with a photodiode series detector (Dionex). The polar metabolites extracted with 75% methanol were separated with a chromatographic column C18 Luna with reversed phase (150 × 2.1 mm, 3µm; Phenomenex) and ionized by electrospray (source ESI) (Fasano et al. New Phytologist 210: 1382-1394). On the contrary, the non-polar metabolites were extracted with acetone, separated with chromatographic column C30 with reversed phase (100 × 3.0 mm; YMC Europe) and ionized by chemical ionization at atmospheric pressure (source APCI).

The identification of metabolites was performed based upon the accurate mass compared to the authentic standards. The mass peak areas were normalized on the mass peak area of the inner standard (formononetin or α-tocopherol acetate) and the abundances of metabolites shown as "fold internal standard" (abundance with respect to the inner standard).

Methods well known to the people skilled in the art may be used to prepare the expression vectors, the recombinant cells and organisms according to the present invention. These methods include in vitro techniques of recombinant DNA, *in vivo* recombination techniques and PCR techniques. See, for example, the techniques described in Maniatis et al., 1989, MOLECULAR CLONING: A LABORATORY MANUAL, Cold Spring Harbor Laboratory, New York; Ausubel et al., 1989, CURRENT PROTOCOLS IN MOLECULAR BIOLOGY, Greene Publishing Associates and Wiley Interscience, New York, and PCR Protocols: A Guide to Methods and Applications (Innis et al., 1990, Academic Press, San Diego, CA).

### DETAILED DESCRIPTION OF THE SEQUENCES

CsALDH1 (SEQ ID NO 1)
   **SEQ. DNA CsALDH1 (1497 bp)** 
ATGGCTGCAACTAACAGCAATGGTATCTTCAAGCTTCCAGAGATCAAGTTCACCAAGCTTTTCAT
CAACGGAGAATTCGTCGATTCCGTCTCCGGGAGAACATTTGAGACAAGGGACCCACGCAATGGC
GACGTTATAGCCAACATCGCAGAAGGAGATAAGGAGGACGTGGATTTGGCGGTGAAGGCAGCC
AGAGAAGCCTTCGACCACGGAAAATGGCCTCGAATGTCTGGTTATGAAAGGGGGAGGATCATG
ATGAAGTTTGCAGATTTGATTGAAGCAAACATAGAAGAATTGGCAGCACTTGACACCTTAGATG
CAGGAAAACTGCTCACTATGGGTAAGGCTGTAGACATCCCAGCAGCAGTACACATGATCCGCTA
CTACGCTGGAGCTGCAGACAAGATACATGGGGAGACACTAAAGTTGTCGAGCGAGTTCCAAGG
ATACACATTAAAGGAGCCCATCGGGGTGGTAGGGCACATAGTTCCCTGGAATTTCCCGACTGCG
ATGTTCGTCATGAAAGTGGGCCCTGCCTTGGCTGCAGGCTGCACAATGATCGTCAAGCCAGCCG
AGCAAACCCCGCTCTCAGCTCTCTACTACGCGCACTTGGCAAAGGAGTCTGGTATTCCCGACGG
AGTAGTCAATGTTGTTACCGGGTATGGCCCAACAGCTGGTGCTGCTCTTTCTTCACACATGGATG
TTGACAAGATTAGTTTTACTGGCTCTACCGAAATAGGACGGGTAGTGATGGAGGCAGCAGCAAA
GAGCAATCTAAAGCACGTATCGCTCGAGTTGGGTGGGAAGTCTCCTCTCATTATTTTTGATGATG
CTAATTTGGACATGGCAGTTAATCTTGCCAGCATGGCCATCTTCTATAACAAGGGAGAAGTTTG
CTGTGCCGGATCTCGTATTTATGTTCAGGAAGGGATATATGATGAGTTTGTGAAGAAGGCAGTG
GAAAAGGCAAAGAGTTGGGTGGTTGGAGACCCCTTTGATCCCAATGTTCAGAATGGGCCTCAGG
TCGACAAGGCACAGTTTGAAAAAGTTCTCAGCTATATTGAGCATGGTAAAAGAGAGGGAGCCA
CCTTGCTGGCAGGAGGAAAAGCCTGTGGTCAGAAAGGCTACTGCATTGAACCAACAATCTTTAC
TGATGTCAAGGAGGATATGAAGATAGCACAAGATGAGATTTTTGGACCAGTCATGTCTCTCATG
AAATTTAAGACAATTGAAGAGGCAATAGAGAAAGCCAACACCACCAGATATGGGTTAGCAGCA
GGAATTGTGACGAATGACTTAAATGTGGCCAACAGCGTATCGAGATCAATTCGTGCCGGCACTG
TTTGGATCAATTGTTACTATGCTTTCGATGCAGAAACTCCCTTTGGCGGTTACAAGATGAGTGGA
TTTGGGAAGGATCAGGGACTCCATGCACTGGAGAAGTACCTCCAAGTGAAGTCTGTGGTCACTC
CTATATATAACTCACCTTGGCTGTGA
**SEQ. AA CsALDH1 (498 aa)** (SEQ ID NO 2) 
MAATNSNGIFKLPEIKFTKLFINGEFVDSVSGRTFETRDPRNGDVIANIAEGDKEDVDLAVKAAREAF
DHGKWPRMSGYERGRIMMKFADLIEANIEELAALDTLDAGKLLTMGKAVDIPAAVHMIRYYAGAA
DKIHGETLKLSSEFQGYTLKEPIGVVGHIVPWNFPTAMFVMKVGPALAAGCTMIVKPAEQTPLSALY
YAHLAKESGIPDGVVNVVTGYGPTAGAALSSHMDVDKISFTGSTEIGRVVMEAAAKSNLKHVSLEL
GGKSPLIIFDDANLDMAVNLASMAIFYNKGEVCCAGSRIYVQEGIYDEFVKKAVEKAKSWVVGDPF
DPNVQNGPQVDKAQFEKVLSYIEHGKREGATLLAGGKACGQKGYCIEPTIFTDVKEDMKIAQDEIFG
PVMSLMKFKTIEEAIEKANTTRYGLAAGIVTNDLNVANSVSRSIRAGTVWINCYYAFDAETPFGGYK
MSGFGKDQGLHALEKYLQVKSVVTPIYNSPWL
CsALDH3 (SEQ ID NO 3)
   **SEQ. DNA CsALDH3 (1554 bp)** 
ATGTCGATGCTACGCGTGGCTCTCTGTCGTCACCGTCTGCTCGGTTCTTCTCGTCACTTCCGCCGA
CAGATGAGTATGGACTCGAAGGGAGCAGTTTCTCAACTGAGCAATTCAGGGATTTTCAGGACTC
AGGGGTTCATTGCAGGGAAATGGCTTGATTCTTATGATGGGACAACCTTTGAGGTGAGAAACCC
TGCTACTGGGGATGTTATAGCAAAAGTGCCATCCATGGGGAGGAAGGAAACTTCAGATGCAAT
ATCTTCGGCGTACACTACTTTGAGTTCTTGGAGCAAACTCACTGCAAATGAAAGGAGCAAATGC
CTCAGAAGATGGTACGATCTAATTATTACTCACAAGGAAGATCTTGCACTACTGATTACATTGG
AACAAGGGAAGCCTCTAACGGAGGCTCAGGGTGAGGTCAGCTATGGTGCCAGCTTCATTGAATT
CTTTGCTGAGGAAGCAAAACGTGTGTATGGCGATATTATTCCTCCTCCTATAGCAGATCGACGA
CTATTTGTTTTGAAACAGCCAATTGGAGTGGTTGCAGCAATTACCCCATGGAACTTTCCTCTGGC
CATGATCGCACGAAAGGTTGGCCCTGCTCTGGCTTGTGGCTGCACAGTGGTTGTTAAGCCTTCAG
AATTTACTCCTTTGACTGCACTGGCTGCAGCTGAGCTCGCTCTTCAAGCTGGAATCCCACCAGGT
GCACTGAACATGGTTATGGGGAAGGCTTCTGATATTGGAGACACTTTATTAGAGAGCACACAGG
TAAGGAAGCTCACTTTCACAGGTTCGACAGCTGTAGGGAAGAAGTTAATGGCAGGATCAGCTAG
TACAGTTAAAAAGGTATCCCTTGAACTTGGTGGGAATGCACCTTGCATAGTCTTTGATGATGCA
AACTTGGATGTAGCAGTGAAAGGCTGTCTTGCTGCGAAGTTTCGAAACAGTGGGCAAACATGCG
TCTGCGCAAACAGAATCCTGGTACAAGAAGGTATCTATGAAAAATTTGCTAATGCATTTGCAAA
GGCTGTTCAAAATCTGCAAGTCGGCAATGGCCTTGCTGAAGGCATAACTCAGGGTCCACTAATT
 
AATGAAGCTGCCGTACAAAAGGTTGAATCGTTTGTCAAAGATGCAACTTCAAAGGGAGCCAATG
TTATACTTGGTGGAAGGAGGCACAGCCTCGGCATGACATTTTATGAGCCTACCGTACTCAGTAA
TGTCAAGAGTGATATGCTCATATCAAGGGAGGAAATATTTGGTCCAGTTGCACCGCTGATGCCT
TTTAAATCTGAAGAAGAAGTTATTCAGATGGCTAATGACACAAATGCTGGTCTAGCCGCTTACA
TATTTACAAACAGCATATCTCGGTCTTGGCGTGTATCTGAAGCTCTTGAATATGGTCTAGTTGGT
GTGAACGAAGGCATAATTTCTACAGAGGTGGCACCATTTGGAGGTTGTAAACAGTCTGGCCTTG
GACGAGAAGGTTCCAAATATGGGATGGATGATTACTTAGAACTCAAATATGTGTGCATGGGGAA
CTTGGAGTCTTGA
**SEQ. AA CsALDH3 (517 aa)** (SEQ ID NO 4) 
MSMLRVALCRHRLLGSSRHFRRQMSMDSKGAVSQLSNSGIFRTQGFIAGKWLDSYDGTTFEVRNPA
TGDVIAKVPSMGRKETSDAISSAYTTLSSWSKLTANERSKCLRRWYDLIITHKEDLALLITLEQGKPL
TEAQGEVSYGASFIEFFAEEAKRVYGDIIPPPIADRRLFVLKQPIGVVAAITPWNFPLAMIARKVGPAL
ACGCTVVVKPSEFTPLTALAAAELALQAGIPPGALNMVMGKASDIGDTLLESTQVRKLTFTGSTAVG
KKLMAGSASTVKKVSLELGGNAPCIVFDDANLDVAVKGCLAAKFRNSGQTCVCANRILVQEGIYEK
FANAFAKAVQNLQVGNGLAEGITQGPLINEAAVQKVESFVKDATSKGANVILGGRRHSLGMTFYEP
TVLSNVKSDMLISREEIFGPVAPLMPFKSEEEVIQMANDTNAGLAAYIFTNSISRSWRVSEALEYGLV
GVNEGIISTEVAPFGGCKQSGLGREGSKYGMDDYLELKYVCMGNLES
CsALDH4 (SEQ ID NO 5)
   **SEQ. DNA CsALDH4 1524 bp)** 
ATGGGATTTACCAAGGAGCACCAGTTCCTCTCCGAGCTCGGGCTCGGCCCGAGAAATCCCGGTT
GCTACGTCGCCGGAAAGTGGAGGGGGAGCGGACCGGTAGTCTCCTCGTCGAATCCCGCGAACA
ATCAGGTTATAGCTGAGGTGGTGGAGGCATCAATGGAAGACTACGAGGATGGCATGAAGGCAT
GTTTTGATGCTTCCAAAATATGGATGCAGATTCCAGCTCCGAAGAGGGGAGAGATTGTTAGACA
GATTGGCGAAGCATTGAGAAGCAAACTCCAGCACCTTGGTAGGCTTGTCTCACTAGAAATGGGA
AAAATTCTTCCCGAAGGGATTGGTGAAGTTCAGGAAATAGTCGACATGTGTGATTATGCTGTTG
GGTTAAGTCGGCAATTGAATGGATCAATCATACCGTCAGAACGTCCGAACCACATGATGATGGA
GGTGTGGAATCCTCTTGGAATTGTAGGTGTTATTACAGCGTTTAACTTCCCGTGTGCTGTGCTTG
GTTGGAATGCCTGCATTGCATTAGTATGTGGAAACTGTGTTGTTTGGAAAGGTGCACCAACAAC
ACCATTAATCACAATTGCTATGACTGAACTAGTTGCTGGGGTGTTGGAGAAGAATAATTTACCA
GGTGCAATTTTCACGTCATTTTGTGGAGGTGCTGAAATTGGCCAAGCAATAAGTCATGACACAC
GGATACCTCTGGTCTCATTTACTGGAAGTTCTAAGGTTGGTCTTATGGTGCAACAAACAGTTAGT
GAGAGATTTGGCAAATGTTTGCTTGAACTTAGTGGAAATAATGCTATAATTGTCATGGATGATG
CTGATATTCAACTTGCTGTACGTTCTGTTCTATTTGCTGCTGTTGGTACAGCTGGCCAGCGATGC
ACAACATGCCGAAGGCTGCTGGTTCACGAAAGCATATATCAAACTGTACTTGATCAATTGGTTG
GAGTCTATAAACAAGTGCAAATTGGAGATCCACTAGAGAAAGGCACCTTATTGGGACCGTTGCA
CACTTCTACTTCAAAGGAGAACTTCGTGAAAGGTGTCCAGGCCATTAAATCCCAGGGAGGGAAG
ATCCTCGTTGGAGGTTCTGTCATAGAATCTGCCGGGAACTTTGTACAACCTACAATTGTCGAGAT
CTCTTCAGATGTACCAATTGTCAAAGAGGAGTTGTTTGGTCCGGTTCTTTATGTGATGAAATTTC
AGACTTTAAAGGAAGCAATTGAAATAAACAACTCTGTACCACAAGGTTTAAGCAGCTCTATATT
CACACGCAAACCAGAAATTATATTCAAGTGGCTGGGACCTCATGGCAGTGATTGTGGAATTGTC
AATGTTAACATACCTACTAATGGTGCTGAAATTGGCGGTGCGTTTGGCGGTGAAAAGGCTACTG
GAGGTGGCCGAGAAGCAGGGAGTGATTCCTGGAAGCAATACATGAGGCGTTCAACTTGTACAA
TCAACTATGGGAGTGAACTCCCTCTGGCACAGGGAATAAACTTTGGCTAG
**SEQ. AA CsALDH4 (507 aa)** (SEQ ID NO 6) 
MGFTKEHQFLSELGLGPRNPGCYVAGKWRGSGPVVSSSNPANNQVIAEVVEASMEDYEDGMKACF
DASKIWMQIPAPKRGEIVRQIGEALRSKLQHLGRLVSLEMGKILPEGIGEVQEIVDMCDYAVGLSRQL
NGSIIPSERPNHMMMEVWNPLGIVGVITAFNFPCAVLGWNACIALVCGNCVVWKGAPTTPLITIAMT
ELVAGVLEKNNLPGAIFTSFCGGAEIGQAISHDTRIPLVSFTGSSKVGLMVQQTVSERFGKCLLELSG
NNAIIVMDDADIQLAVRSVLFAAVGTAGQRCTTCRRLLVHESIYQTVLDQLVGVYKQVQIGDPLEK
GTLLGPLHTSTSKENFVKGVQAIKSQGGKILVGGSVIESAGNFVQPTIVEISSDVPIVKEELFGPVLYV
MKFQTLKEAIEINNSVPQGLSSSIFTRKPEIIFKWLGPHGSDCGIVNVNIPTNGAEIGGAFGGEKATGG
GREAGSDSWKQYMRRSTCTINYGSELPLAQGINFG
CsALDH7 (SEQ ID NO 7)
   **SEQ. DNA CsALDH7 (1449** bp) 
ATGGCCTTCGATGGAGAGAAAGCTAAGGAGATGGTGAAGGAGCTCAGAGAGAGCTTCAACAAA
GGGACGACCAGGAGCTACGAGTGGAGGATGAAGCAGCTGAAGGCCATGGAGAAGATGACTGA
GGAGAAGGAGAAGGATATCATGGATGCCCTCGAATCTGATCTCTCTAAGCCTCAGCTTGAGTCT
TTCCTCCATGAGATTTCAATGGCAAAATCAGTATGTCAGTTTGCTGCTAAGAATTTGAAACGATG
GATGAAACCAGAAAAGGTGCCAGCTCAACTTACAACTTTTCCATCAGTAGGAAATATTGTTGCA
 
GAGCCCTTTGGTGTTGTCCTGATCATCTCAGCATGGAATTTTCCCTTTTTGTTATCTCTCGAACCA
GTCATAGGAGCTATTGCAGCTGGTAATACTGTTGTTCTAAAGCCTTCGGAAATTGCACCTGCCAC
ATCATCATTGTTTGCAAGAATTTTACTTGAGTATGTCGATACCTCATGCGTAAGAGTTGTCGAGG
GCGCTGTTCCTGAAACAACTGCACTTCTAGAGCAGAAGTGGGATAAAATATTCTATACAGGTAA
TGGAAAAGTAGGGCGCGTCGTGATGGCTGCAGCTGCAAAGCACCTAACACCAGTTGTCTTGGAA
CTTGGAGGAAAATGTCCAGTTGTTGTTGATTCAAATATCGATTTAAAAGTTGCGACCAAAAGAG
TTGTTGTTGGGAAGTGGGGATGCAACAATGGACAAGCTTGCATAGCTCCAGATTATATCATAAC
AACAAAATCATTTGCCCCAAAGCTGGTGGAGTCTCTGAAGATCACTTTAGAAAGGTTCTACGGC
GAGGATCCATTGGAAACGGAAGATCTGTCTCGTATTGTAAATGAAAATCATGTTGCGCGTTTGG
CAAGGCTCTTGGATGATGATATGGTTTCTGGCAAGATTATCTATGGTGGAAAGCGAGATGAGAA
GCGACTAAAGATAGCCCCAACACTGTTGTTAGATGTTCCTGACGATTCACTGATTATGAAGGAA
GAGATATTTGGTCCCTTGCTTCCAATTATTACGGTTGACAAGATTGAAGATAGTTTTGCTGTGAT
AAACTCGAAAACGAAGCCTCTTGCTGCTTACCTCTTCACCAAGAACAAGAACCTAGAGAGGATG
TTCGTGGAAACAGTATCTTCCGGAGGCATGCTAATTAACGACACGGTCTTACATGTTGCTAATCC
TTATTTGCCATTTGGTGGAGTTGGTGAGAGCGGAACTGGATCCTACCATGGTAAATTCTCGTTCA
ATGCGTTCAGTCATAAGAAAGCTGTTTTATCCCGTGGATTTGGAGGCGAAGTAGGGGCAAGGTA
TCCTCCTTATACAGATAAGAAGCGAAAGATTATAAGAGCATTGCTTGCGGGTAATATTATAGCC
CTGGTTTTGGCTTTCTTTGGCTTCTCCAAATCTTGA
**SEQ. AA CsALDH7 (482 aa)** (SEQ ID NO 8) 
MAFDGEKAKEMVKELRESFNKGTTRSYEWRMKQLKAMEKMTEEKEKDIMDALESDLSKPQLESFL
HEISMAKSVCQFAAKNLKRWMKPEKVPAQLTTFPSVGNIVAEPFGVVLIISAWNFPFLLSLEPVIGAI
AAGNTVVLKPSEIAPATSSLFARILLEYVDTSCVRVVEGAVPETTALLEQKWDKIFYTGNGKVGRVV
MAAAAKHLTPVVLELGGKCPVVVDSNIDLKVATKRVVVGKWGCNNGQACIAPDYIITTKSFAPKLV
ESLKITLERFYGEDPLETEDLSRIVNENHVARLARLLDDDMVSGKIIYGGKRDEKRLKIAPTLLLDVP
DDSLIMKEEIFGPLLPIITVDKIEDSFAVINSKTKPLAAYLFTKNKNLERMFVETVSSGGMLINDTVLH
VANPYLPFGGVGESGTGSYHGKFSFNAFSHKKAVLSRGFGGEVGARYPPYTDKKRKIIRALLAGNII
ALVLAFFGFSKS
CsUGT2 SEQ ID NO 9)
   **SEQ. DNA CsUGT2 (1380 bp)** 
ATGGAGCAGAAAGATGTGAACGGCAACAAATGCCACATCCTCCTCCCCTGTCCAGCACAAG
GCCATATCAACCCCATTCTCCAATTCGGCAAGCGACTGGCCTCTCACAACCTCCTCACCACACTC
GTCAACACCCGGTTCCTCTCCAACTCGACCAAATCCGAACCCGGTCCGGTCAACATCGAGTGCA
TATCCGACGGATTCGATTCCGGTGGGATGAATGCAGCACCTAGTCGTCGGGCCTATTTCGACCG
ACTCGAGTCGGTCGGGTCAGAAACACTGTTTGGCCTCATCGAGTCACTCCGGTCCAGAGGCCGA
CCGGCGCATGTTTTGGTCTACGACCCGTTCTTGCCTTGGGCAATGAACGTAGCCGAGCGGTCCG
GGCTGCGGTCGGTCGCATTCTTTACTCAGCCGTGTGCAGTGGACACAATCTACCGCCACGTGTG
GGAGGGACGAATTAAGGTTCCGGTTACCGAGCCGGTCCGGTTGCCTGGATTGCCCCCACTCGAG
CCTAGTGATCTGCCGTCGTTCGTAACGGATTCGGACCCAGTCGTTAACCCGGATCTCTTGCCACT
TCTAGTGAATCAGCACAAAAATTTGGACAAAGCTGATATGATGTTGATTAACTCCATATACGAG
CTAGAGCATGAGGAATTTGATTGGATGGAGTCCCGATTACCATTACCGGTGAAGTCGATTGGGC
CGACCGTGCCGTCTACTTATCTCGACAACCGAATCCCGTCGGATTCACACTATGGATTCAATCTA
TACACCCCTGACACTACTCCCTACTTGGACTGGCTCGACTCCAAGGCCCCGAACTCTGTCATCTA
TGTCTCATTTGGTAGTTTGTCCAGTCTGAGCCCTGATCAAACCAATGAGATTGCGTCGGGTCTGA
TCGCCACCAACAAAAGCTTTATTTGGGTGGTACGCACTTCGGAGTTAGCCAAACTCCCTGCCAA
CTTCACCCAAGAGAATGCGAGCCGTGGGCTGGTTGTGACCTGGTGCGACCAACTCGATGTCCTC
GCACACGTTGCGACCGGTTGCTTCGTGACTCACTGCGGATGGAACTCGACTATGGAAGGTATCG
CACTTGGTGTGCCGATGGTGGGAGTGCCGCAGTGGTCGGACCAGCCTATGAATGCCAAGTATGT
AGAGGATGTGTGGAAGGTCGGCGTGAGGGCGAAGACTTATGGGAAGGATTTTGTGAGAGGGGA
GGAGTTCAAAAGATGTGTGGAGGAGGTGATGGATGGAGAGAGGAGTGGAAAGATTAGAGAGA
ATGCCGCAAGGTGGTGTAAGTTGGCCAAGGACTCTGTTAGTGAGGGTGGGAGTTCTGACAAGTG
TATCAAGGAGTTCATACATCAGTGTTGCAAATGA
**SEQ. AA CsUGT2 (459 aa)** (SEQ ID NO 10) 
MEQKDVNGNKCHILLLPCPAQGHINPILQFGKRLASHNLLTTLVNTRFLSNSTKSEPGPV
NIECISDGFDSGGMNAAPSRRAYFDRLESVGSETLFGLIESLRSRGRPAHVLVYDPFLPW
AMNVAERSGLRSVAFFTQPCAVDTIYRHVWEGRIKVPVTEPVRLPGLPPLEPSDLPSFVT
DSDPVVNPDLLPLLVNQHKNLDKADMMLINSIYELEHEEFDWMESRLPLPVKSIGPTVPS
TYLDNRIPSDSHYGFNLYTPDTTPYLDWLDSKAPNSVIYVSFGSLSSLSPDQTNEIASGL
IATNKSFIWVVRTSELAKLPANFTQENASRGLVVTWCDQLDVLAHVATGCFVTHCGWNST
MEGIALGVPMVGVPQWSDQPMNAKYVEDVWKVGVRAKTYGKDFVRGEEFKRCVEEVMDGE
RSGKIRENAARWCKLAKDSVSEGGSSDKCIKEFIHQCCK
CsABCC1 (SEQ ID NO 11)
   **SEQ. DNA CsABCC1 (4491 bp)** 
ATGTCCTCCTCTCCCTACGATCTCACCTCCCTCACTTGCTCACCTCCCCCCACTTCCGACCCAATC
CAATGGCTCCGCTTCGTCGCCCTCTCCCCCTGTCCTCAGCGTCTCCTCCTCTCCGCCGTCGATATT
CTCTTCCTCCTCTCCCTCCTCTTCACCCTCCTAAGAAGACTAATCTTCCTTCCAAACAACAACAAT
AATGAGGCCAGAAAGCCTCTCCTATCCCCGGATGAAGATGACAATGTTCCGTCAGCAAAAGTCA
ATACCAATCTACTCTTCAAGCTCTCCCTCGCTCTCTCCGCCGCCCTTGCCGCCGCCTACCTCGTCC
TATTCGTCGTCGCTTTGGTTCGAATATCCCCACCGACGTGGCGACTCGTCGAGTCGGCCTTCCTA
TTCTTACAATTCCTTACACAATCGTCCGTCGCGATTGTCGTCGGTCACGAGAAGAAGTTCCGCGC
CGAGAAGCACCCGCCGGCTCTCCGCGCCTTCTGGGTCGTCACCTTCGTCGTCGTCTCCCTCTTCT
CCGTCTCCTCCGTCGCTAGGTTCGTCTCGAAGGGTTACGTCTCTCCCGACGATCCCGTCACCGTC
GCCGTCCTCGTCGCGAGCCTGCTCTTCCTGATAACCGCCGTAACCGGCGCGACCGGCATATCGA
CGGGGACGGACCGGCCGGTAATTGCGACGCCGTCGGCGGAGAAGGTGACCGGATACGCGACCG
CATCGCTGCTCTCGCGCGCCACGTGGCACTGGATGAACCCGCTCATGGCGAAGGGATACCGGGA
GCCGATCACGATCGGCGACGTGCCGTCGCTCTCCCCGGAGCACCGGGCGGAGCGGTTGCTGGAG
ACGTTCCGATCCAACTGGCCCGAACCCGCAACCCGGTCCAACCACCCGGTCCGGACCGCGCTGC
TCAAGTCCTTCTGGCCCCACCTCCTCCTCACCGCCGTCCTCTCCATCCTCAAGCTCACCGTCATGT
ACGTGGGGCCCACCCTCATCCAGGGCTTCGTCGACTTCACCTCCGGGGACCGCGCCTCCCCGTA
CGAGGGATTCCGCCTCGTGTTCATTTTGCTGGTGGCGAAGTTCTTCGAGGTGCTGTGCGCGCACC
AGTACAACTTCCAGTCGCAGAAGCTCGGGATGATGATAAGGTCGTGCCTGATCACGTCGCTGTA
CCGGAAGGGCCTGCGGCTATCGTGCGCCGCCAGGCAGGCGCACGGGGTGGGGATGATCGTCAA
CTACATGGCTGTCGACGCGCAACAGCTAGCCGACATGATGTTGCAGGTGCACTACATATGGCTC
ATGCCAGTGCAGGTTAGCGTCGCCATTGCGCTGCTGTATACTTATCTGGGTCCGTCGGTTACGTC
GGCAGTCGCTGGGATCGCTGCGGTGCTCGTGTTCGTCGTGCTGGGGACAAAGAGGAATAATAGG
TATCAGTTTAATTTGATGACTATGAGGGATAAGAGAATGAAGGCAACTAATGAGATGCTTAACT
ATATGAGGGTGATCAAGTTTCAGGCGTGGGAGGAACATTTTGATGAGAGGATCAAGAAGTTCA
GACAAATGGAGTTTGGCTGGCTATCTAGGTTCATGTACTCCATCTCCGGCAACATCATCGCCCTA
TGGAGCGCGCCGGTGCTCGTCTCCTCCCTCGTGTTCGGCACGTGCGTATTCGCTGGGGTCCACCT
CGACGCCGGCCTCGTCTTCACCGCCACCTCATTCTTCAAGATCCTCCAGGAACCCCTCCGGAACT
TCCCCCAAGCAATGATCTCCGCATCCCAAGCGATGATCTCGCTCGACCGGCTCGACTCCTACATG
ACCAGCGGAGAGCTCGACGAGGCGGCGGTGCAGAGAATGGACGGCTGCGATGACGACGTGGCG
ATCGAGTTGAAGGACGGGGTTTTCGGATGGGAGGACAGGGAGGACGCGGAGAAGGAGAAGGA
GAAGGCGTGGCTGAGGGACATAAACATGGAGGTGAAGAGGGGTTCGCTCGCGGCGGTGGTGGG
GACCGTCGGCTCGGGGAAGTCGTCGCTCATCTCGTGCTTGCTCGGCGAGATGCAGAAGATCTCG
GGAACGGTTAGGGTTTGTGGCACTACTGCCTATGTTGCACAGACTGCATGGATTCAGAATGGGA
CTATTGAAGATAACATCCTATTTGGACTGCCGATGGACCGGGAGAAATACAAGGAAGTAATCCG
TGTTTGTTGCTTACAGAAGGACATGGAAATGATGGAGTTTGGGGACCAGACTGAGATTGGTGAG
AGAGGAATAAATCTCAGCGGTGGTCAAAAGCAAAGGATACAGCTGGCTAGGGCTGTGTATCAA
GATTGTGATATCTACCTGCTCGATGATATCTTCAGTGCTGTTGATGCTCACACTGGTTCTGAAAT
CTTCAAGGAATGTGTGAGGGGTGCACTCAGCAAGAAGACGGTCATACTTGTGACTCATCAAGTG
GATTTCTTGCATAATGCTGATCTCATCCTGGTAATGAGAGAAGGGACGATCGTACAATACGGAA
AATACAATGATTTGCTGGGTTCTGGAACAGATTTTGCAGCTCTAGTCGCTGCTCACGACAGTTCA
ATGGAGTTAGTCGAACAGGGTTCTCACAGCAATGAATCCAATAACCCACAGGATAAAGCATCG
GATCAGCCTATCAGGAACTACGGGCAGTCTAACGGGGCAAATGGGTCGATGAACTCGCCCAAG
AAGGAAAAGGGATCGTCAAAGCTCATAAAGGAGGAGGAGAGGGAGACCGGTCATGTCAGCTG
GAATGTTTACAAGAGGTACATCACCGAGGCTTGGGGTTTGTGGGGGCCGCTCACGGTTACCGCT
ATTGCATTTGTGTGGCAGGGTTCGCTAATGTCAAGTGATTACTGGTTGGCATATGAGACATCCGA
AGATAACGCTTCCTCTTTCCGTCCCTCGCTATTTATTGAAGTTTATTCTGGCATTGCCGTTATTTC
GATCTTTCTTGTGGCGGCAAGGTCCTTTCTTATAGCGTATTTGGGGCTGAAGACTGCTCAGATTT
TTTTCAAGCAGATTTTGAACAGTCTCTTGCATGCTCCGATGTCGTTCTTTGACACGACACCTTCA
GGAAGAATTCTCAGTCGGGCATCATCAGATCAAACCAACATCGATCTGTTTCTTCCCTTCTTTGT
GGGGATGACTGTGTCAATGTACATTACAGTGCTTAGTATCATCTTTATTACGTGTCAGAGTGCAT
GGCCAACAATCATAGCCATTTTCCCACTAGGATGGCTGAACATTTGGTACAGGAATTACTATCTC
GCAACATCTCGCGAGCTAACTCGAATCGACTCGATCACGAAAGCACCCGTCATTCATCACTTTTC
AGAGACTATTCTTGGTGTCATGACAATAAGGTGCTTCAGAAAAGAGCAAAGGTTTTCTGAGGAG
AATTTAAAAAGAGTCAATACAAACTTAACCAATGATTTTCACAACAACGGTGCAAATGAGTGGT
TGGGTTTTCGTTTAGAACTGATTGGTTCAGTTGTCTTGTGCATATCTGCTCTTCTCATGGTCATGC
TGCCTAGCAATTTCATTAAACCAGAACTTGTTGGTTTATCTCTTTCTTATGGTCTCTCGCTGAATT
CTGTACTATTTTGGGCGGTATGGATCAGTTGCTTCATAGAGAATAAGATGGTGTTAGTTGAAAG
GATAAAGCAGTTCACCAACATTCCCTCCGAAGCAGCATGGAGGATTGAAGGCTGCCTTCCTACA
CCAAATTGGCCCAATAGAGGAGATATTACTCTGAACGATCTGAAGGTTAGATATCGACCAAATA
CCCCTCTAGTTCTGAATGGCATTACATTGACCATTAAAGGGGGCGAGAAGGTTGGAGTAGTGGG
TCGAACTGGAAGCGGCAAATCGACATTGATACAAGTTCTATTCAGAATTGTAGAGCCTTCTGAG
GGGAAAATAATCATCGATGGAGTAGACATTTGTACACTGGGCCTTCACGATCTCCGGTCACGTT
TTGGCATTATTCCTCAAGAACCCGTTCTCTTTGAAGGAACTGTGAGAAGCAACATCGATCCAATT
GGACAATATTCAGATGAAGATATATGGCAGAGCCTGGATCGTTGCCAACTCAAAGATGCCGTGG
 
CTTCAAAACCTGAAAAACTCGATGCTTTGGTGGTTGATAACGGAGAGAACTGGAGTGTTGGCCA
GAGGCAGCTTTTGTGTTTAGGCCGTGTTATGCTGAAGCGCAGTCGAATTCTATTTATGGACGAAG
CGACTGCTTCCGTGGATTCACAGACCGATGCTGTGATTCAAAAGATCATTCGAGAAGACTTCTC
GGCTTGTACTATTATCAGCATTGCTCATAGAATACCCACAGTCATGGATTGTGATAGAGTGCTGG
TTATCGATGCAGGACTGGCAAAAGAGTTCGACAGCCCAGCCAACTTGATAGATCGGCCATCTCT
CTTTGGCGCATTGGTTCAGGAGTATGCGAACCGCTCTTCTGATCTTTGA
**SEQ. AA CsABCC1 (1496 aa)** (SEQ ID NO 12) 
MSSSPYDLTSLTCSPPPTSDPIQWLRFVALSPCPQRLLLSAVDILFLLSLLFTLLRRLIFLPNNNNNEAR
KPLLSPDEDDNVPSAKVNTNLLFKLSLALSAALAAAYLVLFVVALVRISPPTWRLVESAFLFLQFLTQ
SSVAIVVGHEKKFRAEKHPPALRAFWVVTFVVVSLFSVSSVARFVSKGYVSPDDPVTVAVLVASLLF
LITAVTGATGISTGTDRPVIATPSAEKVTGYATASLLSRATWHWMNPLMAKGYREPITIGDVPSLSPE
HRAERLLETFRSNWPEPATRSNHPVRTALLKSFWPHLLLTAVLSILKLTVMYVGPTLIQGFVDFTSGD
RASPYEGFRLVFILLVAKFFEVLCAHQYNFQSQKLGMMIRSCLITSLYRKGLRLSCAARQAHGVGMI
VNYMAVDAQQLADMMLQVHYIWLMPVQVSVAIALLYTYLGPSVTSAVAGIAAVLVFVVLGTKRN
NRYQFNLM
TMRDKRMKATNEMLNYMRVIKFQAWEEHFDERIKKFRQMEFGWLSRFMYSISGNIIALWSAPVLVS
SLVFGTCVFAGVHLDAGLVFTATSFFKILQEPLRNFPQAMISASQAMISLDRLDSYMTSGELDEAAV
QRMDGCDDDVAIELKDGVFGWEDREDAEKEKEKAWLRDINMEVKRGSLAAVVGTVGSGKSSLISC
LLGEMQKISGTVRVCGTTAYVAQTAWIQNGTIEDNILFGLPMDREKYKEVIRVCCLQKDMEMMEFG
DQTEIGERGINLSGGQKQRIQLARAVYQDCDIYLLDDIFSAVDAHTGSEIFKECVRGALSKKTVILVT
HQVDFLHNADLILVMREGTIVQYGKYNDLLGSGTDFAALVAAHDSSMELVEQGSHSNESNNPQDK
ASDQPIRNYGQSNGANGSMNSPKKEKGSSKLIKEEERETGHVSWNVYKRYITEAWGLWGPLTVTAI
AFVWQGSLMSSDYWLAYETSEDNASSFRPSLFIEVYSGIAVISIFLVAARSFLIAYLGLKTAQIFFKQIL
NSLLHAPMSFFDTTPSGRILSRASSDQTNIDLFLPFFVGMTVSMYITVLSIIFITCQSAWPTIIAIFPLGW
LNIWYRNYYLATSRELTRIDSITKAPVIHHFSETILGVMTIRCFRKEQRFSEENLKRVNTNLTNDFHNN
GANEWLGFRLELIGSVVLCISALLMVMLPSNFIKPELVGLSLSYGLSLNSVLFWAVWISCFIENKMVL
VERIKQFTNIPSEAAWRIEGCLPTPNWPNRGDITLNDLKVRYRPNTPLVLNGITLTIKGGEKVGVVGR
TGSGKSTLIQVLFRIVEPSEGKIIIDGVDICTLGLHDLRSRFGIIPQEPVLFEGTVRSNIDPIGQYSDEDIW
QSLDRCQLKDAVASKPEKLDALVVDNGENWSVGQRQLLCLGRVMLKRSRILFMDEATASVDSQTD
AVIQKIIREDFSACTIISIAHRIPTVMDCDRVLVIDAGLAKEFDSPANLIDRPSLFGALVQEYANRSSDL
CsABCC2 (SEQ ID NO 13)
   **SEQ. DNA CsABCC2 (4881 bp)** 
ATGGGTTTCAAACCGCTTGGTTGGTACTGCCGGCCCGTCAAGGATGGATTGTGGTCAATGACGG
TGCATAATGCTCTGGGTGCCTACACTCCCTGCACCATGGAGACCATTGTGGTTGGTATCTCCCAT
CTGGCTCTTTTTGGCACGTGCTTCTATCGTATATGGCGGATGAGTAGGGATGTAACTGTGAAGAG
ATACTGTTTGAGGTCTAAGTACTACCATATCCTGCTCGGACTGCTTGCTGCTTTCTGCACTTTGG
AGCCTCTCTTCAGGCTGGTGTTCGGTATATCAATCATGGATATCGATCATCACACTAGCCTTCCA
CCATTTGAGATCCTGTCACTGATTGTTGAGGCTGTTGCTTGGTGTTGTATGCTTGTAATGCTTGG
AATGGAAAGTAAGGTTTATATCCATGAATTTCGGTGGTACGTCCGTTTTGTAGTCCTCTATGTCT
TGGTTGGGGAGACTGCTGTTTTTAATCTTGTTCTCTCTGTTAGAGAGTACTATGAGGAATCCGTC
TTTTATCTTTACACCAGCGTGATAGTCTGCCAGTTTTTGTTTGGATTTCTTCTGCTAATTCATATG
CCAACGCTCGATCCGTTCCCTGGATATACTCCCATTAGAGACCAAGTGTTTGTCGATAACACAG
AATACGAGTCCCTTGCTGGAGAAGAGCATATTTGTCCTGAGAGGCATGTCAATTTATTGTCCAA
AATACTCTTTTCCTGGATGACTCCCCTCATGCAACAAGGCTATAGAAAACCTATCACTGAGAGG
GATGTGTGGAAATTAGATTCATGGGATACTACAGAGACGTTGTATAGCAACTTCCAGAAATGCT
GGCTTGAGGAATCCAAGAAACCCAAACCTTGGTTGTTGAGAGCACTACATAGTAGCCTTGGGGG
AAGATTTTGGCTTGGAGGCTTTTTCAAGATTGGCAATGATCTCTCTCAGTTTGTGGGTCCCACGA
TTTTGAATCTCCTCTTACAATCTATGCAAGAAGGGGATCCATCCTGGAATGGCTACATCTACGCA
TTCGCAATTTTTGCTGGAGTGGTATTTGGCGTGCTTTCTGAAGCACAATACTTTCAGAATGTTAT
GCGTGTCGGCTTTCGTTTGAGGTCTACATTGGTTGCTGCAGTATTTCGCAAATCACTAAGATTAA
CACATGAAGGTCGGAGAAAATTTGCATCAGGGAAGATAACTAACTTGATGACAACTGACGCAG
AATCACTGCAGCAAATATGCCAACAGCTTCACAGTTTATGGTCAGCTCCTTTCAGAATCATTTTG
TCTATTATCCTTCTCTACCAGGAGCTTGGTGTTGCTTCACTAGCAGGCGCACTGATGCTTGTTCTC
ATGTTTCCAATACAAACATTGGTGATAAGCAAAATGCAGAAATTAACTAAGGAGGGTATTCAAC
GCACTGACAAGAGAATTGGTCTAATGAATGAGTTATTGGCTGCTATGGACACTGTGAAATGCTA
TGCTTGGGAGCAGAGCTTCCAGTCTAAAGTGCAAAGTATTCGCGATGATGAACTTTCTTGGTTCC
GCAAATCCCAGCTTTTAGGATCGTTGAATTCCTTTATGCTGAATAGCATTCCAGTTGTGGTCACG
GTGGTTTCTTTTGGAGTTTATTCCTTACTTGGAGGGAACTTGACACCTGCAAAGGCATTCACATC
ACTTTCACTGTTTGCAGTACTGCGGTTTCCTCTGTTCATGCTTCCAAATTTGATAACTCAGGTTGT
GAACACAAAAGTCTCACTAGATAGGTTAGAGGGTCTATTTTTGGCCGAGGAGAGAATACTGTTG
CCAAATCCACCTCTTGACCCAACCCTTCCAGCCATCTCAATTAAGGATGGATACTTCTCGTGGGA
TCGGAAGGAGGAGAGGCCCACGTTATCAAATATTAACTTGGATATACCTGTTGGAAGCTTGGTT
GCCATTGTTGGAAGCACTGGAGAAGGAAAAACGTCACTTGTGTCGGCAATGATTGGTGAGCTAC
 
CACCAAAACCAGAAACAAACACAAATGTTACTATTCGAGGAAGTGTTGCCTACGTTCCCCAAGT
TTCATGGATATTCAATGCAACTGTACGGGACAACATATTGTTTGGATCTCCATTTCAACCATTAC
GCTATGAGGAGGCAATCAAGGTGACTGCTTTGCAGCATGATCTAGATACACTTCCGGGTGGTGA
CCTCACGGAGATTGGTGAAAGAGGTGTAAATATCAGTGGTGGACAAAAGCAAAGAGTATCCAT
GGCAAGGGCTGTGTATTCTGATTCTGATGTATATATATTTGATGATCCTTTGAGTGCTTTAGATG
CCCATGTTGGTAGAGAGGTTTTTGAACAGTGTATTGAGGATAAACTTAGAGGGAAAACACGTGT
TCTTGTTACAAACCAGCTACATTTTCTGCCAAATGTGGATAGAATTATTGTTGTTCATGAAGGTA
TGGTTAAAGAGGAGGGCACCTTTGAAGAGCTTAGTAACAACGGTTTGCTTTTCCAGAAGTTAAT
GGAAAATGCTGGAAAAATGGAGGAGCAAATAGAAGAGAAGAAAACCGAAACCAATGACAAAG
AGAGCTCAAAATTGATTGAAAATGGAGAAACTATGAAAACTGAAAATGGCTTGTTGAAGAGTG
AACAAAAAGGGAATAATGGAAAAGAAGGAAAGACTGTACTTATCAAACAGGAGGAACGTGAA
ACAGGTGTTATAAGTTCAAAAGTTCTCCGCAGATACAAGAACGCATTGGGTGGGATATGGGTTG
TTGCGGTTCTCTTTACATGTTATATACTCACAGAAGTTCTTAGAATCTCTAGCAGCACCTGGCTA
AGTTCTTGGACAGATCAGAGCTCAACAAAGACCCATGGGCCAGGATATTATAATCTGATCTATG
CACTTCTTTCTTTTGGTCAGGTAATGGTGACTCTGGTAAACTCCTATTGGTTAATTATTAACAGTC
TTCATGCTGCCAAGAGATTGCATGATGCCATGCTGAACTCTATATTGCGAGCTCCGATGGTCTTC
TTTCATACCAATCCACTTGGGCGGATAATAAACAGATTTGCCAAAGATTTAGGTGATATTGATC
GCAATGTTGCTGTTTTTGTGAGCATGTTTCTGCAACAAGTCATGCAGCTCCTTTCGACTTTTGTAC
TTATAGGCTTTGTCAGCACTTTATCGCTGTGGGCCATCATGCCACTGCTGATTTTATTCTATGCAG
CCTATTTGTATTACCAGAGTACTGCACGTGAAGTAAAGCGGTTAGATTCTATCACAAGATCTCCT
GTCTATGCACAATTTGGAGAAGCATTAAATGGTCTGTCTACAATCCGTGCATACAAGGCATATG
ATAGGATGGCTGCAATTAATGGGAAATCAATGGACAATAATATCAGATATACGCTTGTGAATAT
GGGTGGAAATCGATGGCTTGCTATCCGTTTGGAAACATTGGGGGGCATTATGATTTGGCTTACA
GCAACATTTGCTGTCATGCAAAACCAACGTGCAGAAAACCAGCAGGCATTTGCATCAACAATGG
GTCTTCTTCTTTCTTATGCTTTAAATATTACCAGCTTATTGACTGCTGTGTTGCGGCTTGCAAGTA
TTGCTGAGAATAGTTTGAATGCTGTTGAGCGAATTGGGACTTACATAGAATTGCCTTCTGAGGCT
CCACTTGTTATTGAAGATAACAAACCTCCTCCAGGTTGGCCATCATCAGGTGTTATCAAATTTCA
AGATGTAGTTCTTCGATATAGACCAGAACTTCCTGCTGTTCTTCATGGAATATCCTTCACAATTG
CTGGAAGTGAGAAGATTGGAATAGTTGGGAGGACAGGTGCTGGAAAGTCTAGTATGCTTAATG
CATTGTTTCGGATTGTGGAACTGGAAAGAGGAACAATTTTTATTGATGAGTACGATGTTTCGAA
ATTTGGTCTTTTGGATTTACGGAAAGCTCTGGGGATAATTCCTCAATCACCTGTTCTGTTTTCTGG
GACTGTCCGTTTTAATCTGGACCCCTTCAATGAGCATAATGATGCAGATCTTTGGGAAGCTTTGG
AGAGAGCACATCTAAAAGATGTCATACGGAGGAATGCACTGGGGCTTGATGCTGAGGTTGCAG
AAGCAGGGGAAAATTTTAGTGTTGGACAAAGACAGCTACTGAGTCTTGCTCGTGCATTATTGAG
GAGATCAAAAATCCTTGTACTTGATGAAGCAACAGCAGCTGTTGATGTCCAAACTGATGCCCTT
ATCCAGAAAACAATCAGAGAAGAATTCAAGTCATGCACGATGCTCATTATAGCGCATCGTTTGA
ACACTATAATTGATTGTGACAAGGTTTTACTCCTTGAAGGTGGCCAGGTACTAGAATTTGACAG
ACCAGAAAATCTTCTTTCGAATGAAGATAGTGCTTTCTCCAAAATGGTCCAGAGCACTGGAGCA
GCCAATGCTCAATATTTAAGGGGTTTGGCCGTTGCGGACCGATCAAACAAGGAAGAGACCAGC
AGGCAAGATGGCCAAAGGAGGTGGCTTGCCTCTATTCGCTGGGCAGCAGCTGCTCAATATGCAC
TTGCCCAGAGCCTAACATCTTCACATAATGACCTCCAGAGATTGGAGATTGAGGATGAGAATAG
CATTCTCAAGAAAACAAAGGATGCCGTTATCACCTTGCAGAACGTTTTGGAAGGAAAGCACGAT
TCAGTGATTGAAGAGAGCTTGAATCAGTATCAAGTCCCCAGAGAGAGATGGTGGTCATCTCTAT
ATAAAATGATTGAAGGTCTTGCGTTGATGAGTAGATTGGCCCATAACAGGCTTCAACATCCAGA
ATTTGTGTTTGAAGATCGTTCACTTGATTGGGACAATGCTGAAATGTAG
**SEQ. AA CsABCC2(1626 aa)** (SEQ ID NO 14)

MGFKPLGWYCRPVKDGLWSMTVHNALGAYTPCTMETIVVGISHLALFGTCFYRIWRMSRDVTVKR
YCLRSKYYHILLGLLAAFCTLEPLFRLVFGISIMDIDHHTSLPPFEILSLIVEAVAWCCMLVMLGMESK
VYIHEFRWYVRFVVLYVLVGETAVFNLVLSVREYYEESVFYLYTSVIVCQFLFGFLLLIHMPTLDPFP
GYTPIRDQVFVDNTEYESLAGEEHICPERHVNLLSKILFSWMTPLMQQGYRKPITERDVWKLDSWDT
TETLYSNFQKCWLEESKKPKPWLLRALHSSLGGRFWLGGFFKIGNDLSQFVGPTILNLLLQSMQEGD
PSWNGYIYAFAIFAGVVFGVLSEAQYFQNVMRVGFRLRSTLVAAVFRKSLRLTHEGRRKFASGKITN
LMTTDAESLQQICQQLHSLWSAPFRIILSIILLYQELGVASLAGALMLVLMFPIQTLVISKMQKLTKEG
IQRTDKRI
GLMNELLAAMDTVKCYAWEQSFQSKVQSIRDDELSWFRKSQLLGSLNSFMLNSIPVVVTVVSFGVY
SLLGGNLTPAKAFTSLSLFAVLRFPLFMLPNLITQVVNTKVSLDRLEGLFLAEERILLPNPPLDPTLPAI
SIKDGYFSWDRKEERPTLSNINLDIPVGSLVAIVGSTGEGKTSLVSAMIGELPPKPETNTNVTIRGSVA
YVPQVSWIFNATVRDNILFGSPFQPLRYEEAIKVTALQHDLDTLPGGDLTEIGERGVNISGGQKQRVS
MARAVYSDSDVYIFDDPLSALDAHVGREVFEQCIEDKLRGKTRVLVTNQLHFLPNVDRIIVVHEGM
VKEEGTFEELSNNGLLFQKLMENAGKMEEQIEEKKTETNDKESSKLIENGETMKTENGLLKSEQKG
NNGKEGKTVLIKQEERETGVISSKVLRRYKNALGGIWVVAVLFTCYILTEVLRISSSTWLSSWTDQSS
TKTHGPGYYNLIYALLSFGQVMVTLVNSYWLIINSLHAAKRLHDAMLNSILRAPMVFFHTNPLGRII
NRFAKDLGDIDRNVAVFVSMFLQQVMQLLSTFVLIGFVSTLSLWAIMPLLILFYAAYLYYQSTAREV
KRLDSITRSPVYAQFGEALNGLSTIRAYKAYDRMAAINGKSMDNNIRYTLVNMGGNRWLAIRLETL
GGIMIWLTATFAVMQNQRAENQQAFASTMGLLLSYALNITSLLTAVLRLASIAENSLNAVERIGTYIE

LPSEAPLVIEDNKPPPGWPSSGVIKFQDVVLRYRPELPAVLHGISFTIAGSEKIGIVGRTGAGKSSMLN
ALFRIVELERGTIFIDEYDVSKFGLLDLRKALGIIPQSPVLFSGTVRFNLDPFNEHNDADLWEALERAH
LKDVIRRNALGLDAEVAEAGENFSVGQRQLLSLARALLRRSKILVLDEATAAVDVQTDALIQKTIRE
EFKSCTMLIIAHRLNTIIDCDKVLLLEGGQVLEFDRPENLLSNEDSAFSKMVQSTGAANAQYLRGLA
VADRSNKEETSRQDGQRRWLASIRWAAAAQYALAQSLTSSHNDLQRLEIEDENSILKKTKDAVITLQ
NVLEGKHDSVIEESLNQYQVPRERWWSSLYKMIEGLALMSRLAHNRLQHPEFVFEDRSLDWDNAE
M

## Claims

1. An amino acid sequence having a sequence selected between SEQ ID NO 12 (CsABCC1), SEQ ID NO 14 (CsABCC2) or an amino acid sequence with an identity percentage equal or greater than 85% with SEQ ID NO 12 (CsABCC1) or SEQ ID NO 14 (CsABCC2), wherein said amino acid sequence is a transport protein apt to transport crocins through a lipid membrane.

2. The amino acid sequence according to claim 1 wherein said sequence is SEQ ID NO 12 (CsABCC1) or SEQ ID NO 14 (CsABCC2).

3. A nucleotide sequence encoding a transport protein having amino acid sequence SEQ ID NO 12 (CsABCC1), SEQ ID NO 14 (CsABCC2) or an amino acid sequence with an identity percentage equal or greater than 85% with SEQ ID NO 12 (CsABCC1) or SEQ ID NO 14 (CsABCC2) apt to transport crocins through a lipid membrane.

4. The nucleotide sequence according to claim 3 wherein said nucleotide sequence is SEQ ID NO 11 or SEQ ID NO 13.

5. An expression vector comprising:
- a nucleotide sequence encoding a transport protein having amino acid sequence SEQ ID NO 12 (CsABCC1), SEQ ID NO 14 (CsABCC2) or an amino acid sequence with an identity percentage equal or greater than 85 % with SEQ ID NO 12 (CsABCC1) or SEQ ID NO 14 (CsABCC2) apt to transport crocins through a lipid membrane.

6. The expression vector according to claim 5 wherein said nucleotide sequence is SEQ ID NO 11 or SEQ ID NO 13.

7. The expression vector according to claim 5 or 6 wherein said vector is selected from pNEV, pAG, pYES (Thermo Fisher) vectors for yeast expression; vectors derived from pBI121 (Clontech), or from pCAMBIA (Change) for the expression in plant.

8. A recombinant cell comprising a nucleotide sequence coding for:
- a transport protein having amino acid sequence SEQ ID NO 12 (CsABCC1), SEQ ID NO 14 (CsABCC2) or an amino acid sequence with an identity percentage equal or greater than 85 % with SEQ ID NO 12 (CsABCC1) or SEQ ID NO 14 (CsABCC2).

9. The recombinant cell according to claim 8 wherein said nucleotide sequence is SEQ ID NO 11 or SEQ ID NO 13.

10. The recombinant cell according to claim 8 or 9 wherein said cell is selected from yeast cell, plant cells.

11. A recombinant host organism comprising a nucleotide sequence coding for:
- a transport protein having amino acid sequence SEQ ID NO 12 (CsABCC1), SEQ ID NO 14 (CsABCC2) or an amino acid sequence with an identity percentage equal to or greater than 85% with SEQ ID NO 12 (CsABCC1) or SEQ ID NO 14 (CsABCC2).

12. The recombinant host organism according to claim 11 selected from a microorganism or a plant.

13. The recombinant host organism according to claim 11 wherein said organism is selected from *Saccharomyces cerevisiae, Schizosaccharomyces pombe, Yarrowia lipolytica, Candida glabrata, Ashbya gossypii, Cyberlindnera jadinii, Pichia pastoris, Kluyveromyces lactis, Hansenula polymorpha, Candida boidinii, Arxula adeninivorans, Xanthophyllomyces dendrorhous, Candida albicans species, Arabidopsis thaliana, Nicotiana benthamiana, Zea mays, Solanum lycopersicum, Solanum tuberosum, Oryza sativa*

14. The recombinant host organism according to anyone of claims 11 to 13 wherein said nucleotide sequence is SEQ ID NO 11 or SEQ ID NO 13.

15. A method for accumulating crocins in the vacuole of a host organism comprising a step of expression of a nucleotide sequence encoding a transport protein having amino acid sequence SEQ ID NO 12 (CsABCC1) or SEQ ID NO 14 (CsABCC2).

16. The method according to claim 15 wherein said host organism is a yeast or a recombinant plant.

17. The method according to claim 15 wherein said host organism is a recombinant organism selected from *Saccharomyces cerevisiae, Schizosaccharomyces pombe, Yarrowia lipolytica, Candida glabrata, Ashbya gossypii, Cyberlindnera jadinii, Pichia pastoris, Kluyveromyces lactis, Hansenula polymorpha, Candida boidinii, Arxula adeninivorans, Xanthophyllomyces dendrorhous, Candida albicans species, Arabidopsis thaliana, Nicotiana benthamiana, Zea mays, Solanum lycopersicum, Solanum tuberosum, Oryza sativa.*

18. A use of a protein having SEQ ID NO 12 (CsABCC1) or SEQ ID NO 14 (CsABCC2) to transport crocins through a lipid membrane of a plant or a microorganism, in particular for accumulating crocin in the vacuole.

19. The method according to claim 15, wherein the transportation in the vacuole is monitored through exposition of a preparation of one-extract purified vacuoles containing plant metabolites, wherein said preparation of vacuoles comprises one or more vesicular transporters.

20. The method according to claim 19 wherein said preparation is constituted by yeast microsomes.

21. The method according to claim 19 or 20 wherein said plant extract is an extract obtained with a polar or apolar solvent.

22. The method according to anyone of claims 19 to 21, wherein said vesicular transporters have a sequence selected from SEQ ID NO 12 (CsABCC1), SEQ ID NO 14 (CsABCC2) or an amino acid sequence with an identity percentage equal to or greater than 85% with SEQ ID NO 12 (CsABCC1) or SEQ ID NO 14 (CsABC2) apt to transport crocins through a lipid membrane.

## Patentansprüche

1. Aminosäuresequenz, die eine Sequenz aufweist, die aus SEQ ID NO 12 (CsABCC1), SEQ ID NO 14 (CsABCC2) oder einer Aminosäuresequenz mit einem Identitätsprozentsatz von gleich oder größer als 85 % mit SEQ ID NO 12 (CsABCC1) oder SEQ ID NO 14 (CsABCC2) ausgewählt ist, wobei die Aminosäuresequenz ein Transportprotein ist, das geeignet ist, Krozine durch eine Lipidmembran zu transportieren.

2. Aminosäuresequenz nach Anspruch 1, wobei die Sequenz SEQ ID NO 12 (CsABCC1) oder SEQ ID NO 14 (CsABCC2) ist.

3. Nukleotidsequenz, die für ein Transportprotein kodiert, das die Aminosäuresequenz SEQ ID NO 12 (CsABCC1), SEQ ID NO 14 (CsABCC2) oder eine Aminosäuresequenz mit einem Identitätsprozentsatz von gleich oder mehr als 85 % mit SEQ ID NO 12 (CsABCC1) oder SEQ ID NO 14 (CsABCC2) aufweist, die geeignet ist, Krozine durch eine Lipidmembran zu transportieren.

4. Nukleotidsequenz nach Anspruch 3, wobei die Nukleotidsequenz SEQ ID NO 11 oder SEQ ID NO 13 ist.

5. Expressionsvektor, umfassend:
- eine Nukleotidsequenz, die für ein Transportprotein kodiert, das die Aminosäuresequenz SEQ ID NO 12 (CsABCC1), SEQ ID NO 14 (CsABCC2) oder eine Aminosäuresequenz mit einem Identitätsprozentsatz von gleich oder mehr als 85 % mit SEQ ID NO 12 (CsABCC1) oder SEQ ID NO 14 (CsABCC2) aufweist, das geeignet ist, um Krozine durch eine Lipidmembran zu transportieren.

6. Expressionsvektor nach Anspruch 5, wobei die Nukleotidsequenz SEQ ID NO 11 oder SEQ ID NO 13 ist.

7. Expressionsvektor nach Anspruch 5 oder 6, wobei der Vektor aus pNEV-, pAG-, pYES(Thermo Fisher)-Vektoren für eine Hefeexpression; Vektoren, die von pBI121 (Clontech) oder von pCAMBIA (Change) für die Expression in Pflanzen abgeleitet sind, ausgewählt ist.

8. Rekombinante Zelle, umfassend eine Nukleotidsequenz, die kodiert für:
- ein Transportprotein, das die Aminosäuresequenz SEQ ID NO 12 (CsABCC1), SEQ ID NO 14 (CsABCC2) oder eine Aminosäuresequenz mit einem Identitätsprozentsatz gleich oder größer als 85 % mit SEQ ID NO 12 (CsABCC1) oder SEQ ID NO 14 (CsABCC2) aufweist.

9. Rekombinante Zelle nach Anspruch 8, wobei die Nukleotidsequenz SEQ ID NO 11 oder SEQ ID NO 13 ist.

10. Rekombinante Zelle nach Anspruch 8 oder 9, wobei die Zelle aus Hefezellen, Pflanzenzellen ausgewählt ist.

11. Rekombinanter Wirtsorganismus, umfassend eine Nukleotidsequenz, die kodiert für:
- ein Transportprotein, das die Aminosäuresequenz SEQ ID NO 12 (CsABCC1), SEQ ID NO 14 (CsABCC2) oder eine Aminosäuresequenz mit einem Identitätsprozentsatz gleich oder größer als 85 % mit SEQ ID NO 12 (CsABCC1) oder SEQ ID NO 14 (CsABCC2) aufweist.

12. Rekombinanter Wirtsorganismus nach Anspruch 11, der aus einem Mikroorganismus oder einer Pflanze ausgewählt ist.

13. Rekombinanter Wirtsorganismus nach Anspruch 11, wobei der Organismus aus *Saccharomyces cerevisiae, Schizosaccharomyces pombe, Yarrowia lipolytica, Candida glabrata, Ashbya gossypii, Cyberlindnera jadinii, Pichia pastoris, Kluyveromyces lactis, Hansenula polymorpha, Candida boidinii, Arxula adeninivorans, Xanthophyllomyces dendrorhous, Candida albicans species, Arabidopsis thaliana, Nicotiana benthamiana, Zea mays, Solanum lycopersicum, Solanum tuberosum, Oryza sativa* ausgewählt ist.

14. Rekombinanter Wirtsorganismus nach einem der Ansprüche 11 bis 13, wobei die Nukleotidsequenz SEQ ID NO 11 oder SEQ ID NO 13 ist.

15. Verfahren zum Akkumulieren von Krozinen in der Vakuole eines Wirtsorganismus, umfassend einen Schritt der Expression einer Nucleotidsequenz, die für ein Transportprotein kodiert, das die Aminosäuresequenz SEQ ID NO 12 (CsABCC1) oder SEQ ID NO 14 (CsABCC2) aufweist.

16. Verfahren nach Anspruch 15, wobei der Wirtsorganismus eine Hefe oder eine rekombinante Pflanze ist.

17. Verfahren nach Anspruch 15, wobei der Wirtsorganismus ein rekombinanter Organismus ist, der aus *Saccharomyces cerevisiae, Schizosaccharomyces pombe, Yarrowia lipolytica, Candida glabrata, Ashbya gossypii, Cyberlindnera jadinii, Pichia pastoris, Kluyveromyces lactis, Hansenula polymorpha, Candida boidinii, Arxula adeninivorans, Xanthophyllomyces dendrorhous, Candida albicans species, Arabidopsis thaliana, Nicotiana benthamiana, Zea mays, Solanum lycopersicum, Solanum tuberosum, Oryza sativa* ausgewählt ist.

18. Verwendung eines Proteins, das SEQ ID NO 12 (CsABCC1) oder SEQ ID NO 14 (CsABCC2) aufweist, um Krozine durch eine Lipidmembran einer Pflanze oder eines Mikroorganismus zu transportieren, insbesondere zum Akkumulieren von Krozin in der Vakuole.

19. Verfahren nach Anspruch 15, wobei der Transport in der Vakuole durch Exposition eine Zubereitung aus gereinigten Vakuolen mit einem Extrakt, das Pflanzenmetaboliten enthält, überwacht wird, wobei die Zubereitung aus Vakuolen einen oder mehrere vesikuläre Transporter umfasst.

20. Verfahren nach Anspruch 19, wobei die Zubereitung aus Hefemikrosomen besteht.

21. Verfahren nach Anspruch 19 oder 20, wobei der Pflanzenextrakt ein Extrakt ist, das mit einem polaren oder apolaren Lösungsmittel gewonnen wird.

22. Verfahren nach einem der Ansprüche 19 bis 21, wobei die vesikulären Transporter eine Sequenz aufweisen, die aus SEQ ID NO 12 (CsABCC1), SEQ ID NO 14 (CsABCC2) oder einer Aminosäuresequenz mit einem Identitätsprozentsatz gleich oder größer als 85 % mit SEQ ID NO 12 (CsABCC1) oder SEQ ID NO 14 (CsABC2) ausgewählt ist, die geeignet ist, um Krozine durch eine Lipidmembran zu transportieren.

## Revendications

1. Séquence d'acides aminés ayant une séquence choisie entre SEQ ID NO 12 (CsABCC1), SEQ ID NO 14 (CsABCC2) ou une séquence d'acides aminés avec un pourcentage d'identité égal ou supérieur à 85 % avec SEQ ID NO 12 (CsABCC1) ou SEQ ID NO 14 (CsABCC2), dans laquelle ladite séquence d'acides aminés est une protéine de transport apte à transporter des crocines à travers une membrane lipidique.

2. Séquence d'acides aminés selon la revendication 1, dans laquelle ladite séquence est SEQ ID NO 12 (CsABCC1) ou SEQ ID NO 14 (CsABCC2).

3. Séquence de nucléotides codant pour une protéine de transport ayant la séquence d'acides aminés SEQ ID NO 12 (CsABCC1), SEQ ID NO 14 (CsABCC2) ou une séquence d'acides aminés avec un pourcentage d'identité égal ou supérieur à 85 % avec SEQ ID NO 12 (CsABCC1) ou SEQ ID NO 14 (CsABCC2), apte à transporter des crocines à travers une membrane lipidique.

4. Séquence de nucléotides selon la revendication 3, dans laquelle ladite séquence de nucléotides est SEQ ID NO 11 ou SEQ ID NO 13.

5. Vecteur d'expression comprenant :
- une séquence de nucléotides codant pour une protéine de transport ayant la séquence d'acides aminés SEQ ID NO 12 (CsABCC1), SEQ ID NO 14 (CsABCC2) ou une séquence d'acides aminés avec un pourcentage d'identité égal ou supérieur à 85 % avec SEQ ID NO 12 (CsABCC1) ou SEQ ID NO 14 (CsABCC2), apte à transporter des crocines à travers une membrane lipidique.

6. Vecteur d'expression selon la revendication 5, dans lequel ladite séquence de nucléotides est SEQ ID NO 11 ou SEQ ID NO 13.

7. Vecteur d'expression selon la revendication 5 ou 6, dans lequel ledit vecteur est choisi parmi les vecteurs pNEV, pAG, pYES (Thermo Fisher) pour l'expression de la levure ; des vecteurs dérivés de pBI121 (Clontech), ou de pCAMBIA (Change) pour l'expression dans la plante.

8. Cellule recombinante comprenant une séquence de nucléotides codant pour :
- une protéine de transport ayant la séquence d'acides aminés SEQ ID NO 12 (CsABCC1), SEQ ID NO 14 (CsABCC2) ou une séquence d'acides aminés avec un pourcentage d'identité égal ou supérieur à 85 % avec SEQ ID NO 12 (CsABCC1) ou SEQ ID NO 14 (CsABCC2).

9. Cellule recombinante selon la revendication 8, dans laquelle ladite séquence de nucléotides est SEQ ID NO 11 ou SEQ ID NO 13.

10. Cellule recombinante selon la revendication 8 ou 9, dans laquelle ladite cellule est choisie parmi les cellules de levure, les cellules végétales.

11. Organisme hôte recombinant comprenant une séquence de nucléotides codant pour :
- une protéine de transport ayant la séquence d'acides aminés SEQ ID NO 12 (CsABCC1), SEQ ID NO 14 (CsABCC2) ou une séquence d'acides aminés avec un pourcentage d'identité égal ou supérieur à 85 % avec SEQ ID NO 12 (CsABCC1) ou SEQ ID NO 14 (CsABCC2).

12. Organisme hôte recombinant selon la revendication 11 choisi parmi un micro-organisme ou une plante.

13. Organisme hôte recombinant selon la revendication 11, dans lequel ledit organisme est choisi parmi *Saccharomyces cerevisiae, Schizosaccharomyces pombe, Yarrowia lipolytica, Candida glabrata, Ashbya gossypii, Cyberlindnera jadinii, Pichia pastoris, Kluyveromyces lactis, Hansenula polymorpha, Candida boidinii, Arxula adeninivorans, Xanthophyllomyces dendrorhous, Candida albicans species, Arabidopsis thaliana, Nicotiana benthamiana, Zea mays, Solanum lycopersicum, Solanum tuberosum, Oryza sativa*

14. Organisme hôte recombinant selon l'une quelconque des revendications 11 à 13, dans lequel ladite séquence de nucléotides est SEQ ID NO 11 ou SEQ ID NO 13.

15. Méthode d'accumulation de crocines dans la vacuole d'un organisme hôte comprenant une étape d'expression d'une séquence de nucléotides codant pour une protéine de transport présentant la séquence d'acides aminés SEQ ID NO 12 (CsABCC1) ou SEQ ID NO 14 (CsABCC2).

16. Méthode selon la revendication 15, dans laquelle l'organisme hôte est une levure ou une plante recombinante.

17. Méthode selon la revendication 15, dans laquelle ledit organisme hôte est un organisme recombinant choisi parmi *Saccharomyces cerevisiae, Schizosaccharomyces pombe, Yarrowia lipolytica, Candida glabrata, Ashbya gossypii, Cyberlindnera jadinii, Pichia pastoris, Kluyveromyces lactis, Hansenula polymorpha, Candida boidinii, Arxula adeninivorans, Xanthophyllomyces dendrorhous, Candida albicans species, Arabidopsis thaliana, Nicotiana benthamiana, Zea mays, Solanum lycopersicum, Solanum tuberosum, Oryza sativa.*

18. Utilisation d'une protéine ayant SEQ ID NO 12 (CsABCC1) ou SEQ ID NO 14 (CsABCC2) pour transporter des crocines à travers une membrane lipidique d'une plante ou d'un micro-organisme, en particulier pour accumuler des crocines dans la vacuole.

19. Méthode selon la revendication 15, dans laquelle le transport dans la vacuole est contrôlé par l'exposition d'une préparation de vacuoles purifiées d'un seul extrait contenant des métabolites végétaux, dans laquelle ladite préparation de vacuoles comprend un ou plusieurs transporteurs vésiculaires.

20. Méthode selon la revendication 19, dans laquelle ladite préparation est constituée de microsomes de levure.

21. Procédé selon la revendication 19 ou 20 dans lequel ledit extrait végétal est un extrait obtenu avec un solvant polaire ou apolaire.

22. Méthode selon l'une quelconque des revendications 19 à 21, dans laquelle lesdits transporteurs vésiculaires ont une séquence choisie parmi SEQ ID NO 12 (CsABCC1), SEQ ID NO 14 (CsABCC2) ou une séquence d'acides aminés avec un pourcentage d'identité égal ou supérieur à 85 % avec SEQ ID NO 12 (CsABCC1) ou SEQ ID NO 14 (CsABC2) apte à transporter des crocines à travers une membrane lipidique.
